(19) Europäisches Patentamt
European Patent Office
Office européen des brevets

(11) EP 2 504 096 B1

(12) FASCICULE DE BREVET EUROPEEN

(45) Date de publication et mention de la délivrance du brevet:
**24.07.2019 Bulletin 2019/30**

(21) Numéro de dépôt: **10805445.3**

(22) Date de dépôt: **18.11.2010**

(51) Int Cl.:
***B01J 21/18*** (2006.01) ***B01J 23/06*** (2006.01)
***B01J 23/755*** (2006.01) ***B01J 23/72*** (2006.01)
***C02F 3/32*** (2006.01) ***B09C 1/10*** (2006.01)
***C07C 31/34*** (2006.01)

(86) Numéro de dépôt international:
**PCT/FR2010/052451**

(87) Numéro de publication internationale:
**WO 2011/064487 (03.06.2011 Gazette 2011/22)**

# (54) UTILISATION DE PLANTES ACCUMULATRICES DE METAUX POUR LA PREPARATION DE CATALYSEURS UTILISABLES DANS DES REACTIONS CHIMIQUES.

VERWENDUNG VON METALLSPEICHENDEN PFLANZEN ZUR VORBEREITUNG VON KATALYSATOREN FÜR CHEMISCHE REAKTIONEN

USE OF METAL-EXTRACTING PLANTS FOR PREPARING CATALYSTS USEFUL IN CHEMICAL REACTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.11.2009 PCT/FR2009/052312**

(43) Date de publication de la demande:
**03.10.2012 Bulletin 2012/40**

(60) Demande divisionnaire:
**17182733.0 / 3 260 196**

(73) Titulaires:
- **Centre National de la Recherche Scientifique 75794 Paris Cedex 16 (FR)**
- **Université Montpellier 2 Sciences et Techniques 34000 Montpellier Cedex 5 (FR)**

(72) Inventeurs:
- **GRISON, Claude F-34170 Castelnau-le-Lez (FR)**
- **ESCARRE, José F-34090 Montpellier (FR)**

(74) Mandataire: **Lavoix 2, place d'Estienne d'Orves 75441 Paris Cedex 09 (FR)**

(56) Documents cités:
**EP-A1- 1 806 177** **EP-A1- 2 327 476**
**WO-A1-00/28093** **WO-A1-94/29226**
**WO-A1-97/34714** **WO-A1-2006/096472**
**WO-A1-2011/064462** **WO-A1-2011/064487**
**WO-A1-2013/150197** **WO-A1-2014/016509**
**WO-A2-2007/083304** **GB-A- 1 604 768**
**US-A- 4 525 218** **US-A1- 2005 217 174**
**US-A1- 2008 008 676**

- **PRABHA K PADMAVATHIAMMA ET AL: "Phytoremediation Technology: Hyper-accumulation Metals in Plants", WATER, AIR, AND SOIL POLLUTION, KLUWER ACADEMIC PUBLISHERS, DO LNKD-DOI:10.1007/S11270-007-9401-5, vol. 184, no. 1-4, 22 mai 2007 (2007-05-22), pages 105-126, XP019535063, ISSN: 1573-2932**
- **STALS M ET AL: "Flash pyrolysis of heavy metal contaminated biomass from phytoremediation: Influence of temperature, entrained flow and wood/leaves blended pyrolysis on the behaviour of heavy metals", JOURNAL OF ANALYTICAL AND APPLIED PYROLYSIS, ELSEVIER BV, NL, vol. 87, no. 1, 1 janvier 2010 (2010-01-01), pages 1-7, XP026807617, ISSN: 0165-2370 [extrait le 2009-09-15]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 504 096 B1

- LIEVENS C ET AL: "Study of the potential valorisation of heavy metal contaminated biomass via phytoremediation by fast pyrolysis: Part I. Influence of temperature, biomass species and solid heat carrier on the behaviour of heavy metals", FUEL, IPC SCIENCE AND TECHNOLOGY PRESS, GUILDFORD, GB LNKD-DOI:10.1016/J.FUEL.2007.10.021, vol. 87, no. 10-11, 1 août 2008 (2008-08-01), pages 1894-1905, XP022611182, ISSN: 0016-2361 [extrait le 2007-11-21]
- DATABASE WPI Week 200933 Thomson Scientific, London, GB; AN 2009-G46945 XP002624031, & CN 101 381 351 A (UNIV SOUTH CHINA SCI&ENG) 11 mars 2009 (2009-03-11)
- YANG J G ET AL: "Heavy metal removal and crude bio-oil upgrade from Sedum alfredii Hance harvest using hydrothermal upgrading", JOURNAL OF HAZARDOUS MATERIALS, ELSEVIER, AMSTERDAM, NL, vol. 179, no. 1-3, 15 July 2010 (2010-07-15), pages 1037-1041, XP027044961, ISSN: 0304-3894 [retrieved on 2010-05-11]
- YANG ET AL: "Heavy metal removal and crude bio-oil upgrading from Sedum plumbizincicola harvest using hydrothermal upgrading process", BIORESOURCE TECHNOLOGY, ELSEVIER BV, GB, vol. 101, no. 19, 1 October 2010 (2010-10-01), pages 7653-7657, XP027089417, ISSN: 0960-8524 [retrieved on 2010-05-23]
- HU P J ET AL: "Tolerance, accumulation and distribution of zinc and cadmium in hyperaccumulator Potentilla griffithii", ENVIRONMENTAL AND EXPERIMENTAL BOTANY, ELSEVIER, AMSTERDAM, NL, vol. 66, no. 2, 1 May 2009 (2009-05-01), pages 317-325, XP026161711, ISSN: 0098-8472, DOI: 10.1016/J.ENVEXPBOT.2009.02.014 [retrieved on 2009-03-13]
- ZHANG S ET AL: "A newly found cadmium accumulatorMalva sinensis Cavan", JOURNAL OF HAZARDOUS MATERIALS, ELSEVIER, AMSTERDAM, NL, vol. 173, no. 1-3, 15 January 2010 (2010-01-15), pages 705-709, XP026782519, ISSN: 0304-3894, DOI: 10.1016/J.JHAZMAT.2009.08.142 [retrieved on 2009-09-04]
- TEOFILO VAMERALI ET AL: "Field crops for phytoremediation of metal-contaminated land. A review", ENVIRONMENTAL CHEMISTRY LETTERS, SPRINGER, BERLIN, DE, vol. 8, no. 1, 30 December 2009 (2009-12-30), pages 1-17, XP019765247, ISSN: 1610-3661
- GUILLAUME LOSFELD ET AL: "Design and performance of supported Lewis acid catalysts derived from metal contaminated biomass for FriedelCrafts alkylation and acylation", CATALYSIS TODAY, ELSEVIER, NL, vol. 189, no. 1, 21 February 2012 (2012-02-21), pages 111-116, XP028400049, ISSN: 0920-5861, DOI: 10.1016/J.CATTOD.2012.02.044 [retrieved on 2012-03-05]
- Khaiwal Ravindra ET AL: "Platinum group elements in the environment and their health risk", Science of The Total Environment, vol. 318, no. 1-3, 1 January 2004 (2004-01-01), pages 1-43, XP055112626, ISSN: 0048-9697, DOI: 10.1016/S0048-9697(03)00372-3
- James R. Kastner ET AL: "Low Temperature Catalytic Oxidation of Hydrogen Sulfide and Methanethiol Using Wood and Coal Fly Ash", Environmental Science & Technology, vol. 37, no. 11, 1 June 2003 (2003-06-01), pages 2568-2574, XP055123527, ISSN: 0013-936X, DOI: 10.1021/es0259988
- KOLAR ET AL: "Low temperature catalytic oxidation of aldehydes using wood fly ash and molecular oxygen", APPLIED CATALYSIS B: ENVIRONMENTAL, ELSEVIER, AMSTERDAM, NL, vol. 76, no. 3-4, 29 October 2007 (2007-10-29), pages 203-217, XP022318395, ISSN: 0926-3373

## Description

L'invention concerne l'utilisation de plantes accumulatrices de métaux pour la mise en oeuvre de réactions chimiques.

La décontamination biologique des sols pollués par les métaux, les métalloïdes, les déchets et rejets organiques industriels et agricoles ou les radio-isotopes est un problème très préoccupant car le sol exerce des fonctions essentielles qui déterminent en grande partie la production des produits alimentaires et la qualité de l'eau.

Parmi les différentes substances polluantes, les métaux lourds font partie des composés les plus nocifs, car ils ne sont pas biodégradables et se concentrent dans les sols. L'exemple proche de Saint Laurent Le Minier (Gard) illustre parfaitement l'ampleur du problème. L'exploitation des gisements miniers proches de Ganges de l'époque romaine jusqu'en 1992 (Rolley J.P., la petite histoire du plomb et du Zinc en Cévennes, www.ensm-ales.fr/~jprolley/Geologie/Pb-Zn.html, 2002), a entraîné une contamination importante des sols par le zinc, le plomb, le cadmium (rapport EMETER, Eléments rares métalliques (ETM) dans le continuum sol-plante, espèces tolérantes et restauration des sites industriels, Contrat Ademe, Coordinateur J. Escarré, 2008).

Des situations analogues sont connues en Belgique, au Luxembourg, dans le Jura, les Préalpes suisses ou dans les Pyrénées, pour ne citer que les régions les plus proches ainsi que dans des régions plus éloignées telle que la Nouvelle-Calédonie où le Nickel est plus particulièrement exploité.

Les technologies de dépollution du sol sont difficiles à élaborer, car il s'agit d'un milieu hétérogène, complexe et dynamique, qui joue un rôle clé tampon et de transformateur des polluants.

Différentes techniques de phytoremédiation (phytoextraction, phytodégradation, phytostabilisation, phytostimulation, phytotransformation, phytovolatilisation et rhizofiltration) sont actuellement en plein développement (Terry, N. et Banuelos G., editors, Phytoremediation of contaminated soil in water, Lewis Publishers, Boca Raton, Fl.2000).

Le Centre d'Ecologie Fonctionnelle et Evolutive (CEFE) et plus particulièrement l'équipe du Docteur Escarré étudie la technique de phytostabilisation qui consiste à végétaliser les sols contaminés par des plantes capables de croître en présence de métaux lourds (on parle de tolérance) (Frérot et al., Specific interactions between local metallicolous plants improve the phytostabilazation of mine soils, Plant and Soil, 282 53-65, 2006).Certaines de ces espèces végétales utilisées ont la particularité d'accumuler des métaux en grande quantité dans leurs vacuoles (on parle de plantes hyperaccumulatrices).

L'équipe étudie tout particulièrement deux plantes, l'une *Thlaspi caerulescens* appartenant à la famille des Brassicacées, possède des propriétés remarquables de tolérance et d'hyperaccumulation du zinc, cadmium, nickel. Elle les concentre au niveau des parties aériennes (feuilles et tiges).

Cette plante est capable de stocker le zinc à des concentrations 100 fois supérieures à celle d'une plante classique. Par ailleurs, elle est capable d'extraire et concentrer le zinc et le cadmium dans les tissus aériens, même sur des sols ayant une faible concentration de ces deux métaux.

L'autre plante présente dans le district minier de Saint Laurent Le Minier, susceptible d'accumuler des fortes quantités de zinc, est *Anthyllis vulneraria* : une des très rares légumineuses de la flore des régions tempérées à tolérer et accumuler les métaux. Cette espèce a été déjà utilisée avec succès pour la phytostabilisation du site des Avinières à Saint Laurent Le Minier (Frérot et al., Specific interactions between local metallicolous plants improve the phytostabilisation of mine soils, Plant and Soil, 282 53-65, 2006).

Par ailleurs, il a été montré que si *Anthyllis vulneraria* était également capable de concentrer les métaux lourds dans ses parties aériennes, elle jouait aussi un rôle majeur dans la phytostabilisation des sites pollués en facilitant l'implantation d'autres espèces végétales. Cela est due à la capacité d'*Anthyllis vulneraria* de s'associer avec des bactéries métallicoles appartenant au genre *Mesorhizobium* fixatrices d'azote (Vidal et al., Mesorhizobium metallidurans sp. nov., a novel metal-resistant symbiont of Anthyllis vulneraria, growing on metallicolous soil in Languedoc, France. International Journal of Systematic and Evolutionary Microbiology, sous presse, 2009).

Compte tenu de l'importance de la fixation biologique de l'azote dans la réhabilitation des milieux naturels et plus particulièrement celle des milieux pollués, l'utilisation d'une légumineuse est indispensable pour enrichir rapidement les sols en azote.

La présence *d'Anthyllis vulneraria* permet d'accélérer la colonisation de ces sites par d'autres espèces non fixatrices comme les graminées telles que *Festuca arvernensis,* une autre espèce tolérante mais non accumulatrice de métaux lourds.

Au delà de leur tolérance inhabituelle à $Zn^{2+}$ et $Cd^{2+}$, les plantes hyperaccumulatrices sont capables d'extraire les métaux et les transférer aux parties aériennes où ils se concentrent. De ce fait, les racines contiennent très peu de métaux lourds contrairement aux espèces végétales non accumulatrices. Cette triple propriété de tolérance/accumulation/concentration dans les parties récoltables en fait un outil pertinent en phytoremédiation.

Cependant, certains problèmes restent encore à résoudre si l'on veut dépasser le cadre de la simple stabilisation de sédiments pollués et espérer un développement de la phytoextraction à grande échelle. La valorisation de la biomasse enrichie en métaux lourds reste à développer, car actuellement, seul un transfert des métaux du sol vers la plante est

effectué. Les métaux ne sont pas éliminés du site

Par ailleurs, les métaux lourds sont couramment utilisés en chimie organique comme catalyseurs indispensables à la réalisation de transformations chimiques qui nécessitent une énergie d'activation importante. Le rôle des catalyseurs est alors d'abaisser la barrière énergétique.

Leur mode de fonctionnement repose fréquemment sur leurs propriétés d'acide de Lewis. Le chlorure de zinc fait partie des plus utilisés et est indispensable dans de nombreuses réactions industrielles et de laboratoire. Il est aussi fréquemment utilisé en chimie organique hétérocyclique pour catalyser de nombreuses substitutions électrophiles aromatiques.

Il est également un catalyseur de choix pour réaliser les hydrogénations d'alcools primaires avec le réactif de Lucas, les réactions d'acétalisation, d'aldolisation ou des réactions de cycloadditions de type Diels-Alder...

Ils sont également très utiles en électrochimie analytique, électrométallurgie et extraction liquide-solide où les champs d'application sont nombreux et directement impliqués dans les différents domaines de la vie économique (batteries, piles et accumulateurs, détecteurs d'appareils spectroscopiques, métallurgie, soudures...)

Leur production repose sur des procédés de métallurgie extractive à partir de minerais.

Deux procédés sont possibles (Darcy M., Métallurgie du zinc, 1988, éditions techniques de l'ingénieur ; Philibert J. et al., Métallurgie du minerai au matériau, Editions Dunod, 2ème édition, 2002) :

- la pyrométallurgie qui nécessite des traitements thermiques successifs pouvant dépasser les 1000°C,
- l'hydrométallurgie qui repose sur des traitements acides forts suivis d'une électrolyse très consommatrice d'énergie électrique. Elle possède également un impact environnemental par rejet d'effluents pollués.

La diversité des minerais ne permet pas de disposer de procédés uniques. Bon nombre d'entre eux nécessitent des phases d'extraction liquide-liquide intermédiaires, ce qui entraîne inévitablement l'utilisation de solvants organiques nocifs pour l'environnement et des coûts d'extraction élevés.

La présente invention concerne un procédé selon la revendication 1.

L'un des aspects de l'invention concerne l'utilisation de catalyseurs métalliques provenant de plantes accumulatrices métaux lourds évitant l'emploi de solvants organiques nocifs pour l'environnement et le rejet d'effluents pollués, et permettant l'élimination des métaux lourds des sites pollués par ceux-ci et la valorisation de la biomasse les contenant.

Un autre aspect consiste à fournir des procédés chimiques mettant en oeuvre de tels catalyseurs.

L'expression « plante calcinée ou partie de plante calcinée ayant accumulé au moins un métal» désigne en premier lieu toutes les parties aériennes (feuilles, tiges...) de la plante dans lesquelles les métaux, préalablement présents dans un sol contaminé par ceux-ci, se sont accumulés, c'est-à-dire ont été stockés, notamment dans les vacuoles des plantes, par exemple sous forme de carboxylates métalliques, notamment de malate métallique majoritairement, mais également de citrate, succinate et oxalate. Ils peuvent également être stockés associés à des acides aminés de protéines chélatantes, phytochélatines ou métallothionéines.

Le terme « calcinée » désigne un traitement thermique de la plante, notamment de 200°C à 400°C, en particulier 300°C, permettant de déshydrater la plante et de détruire au moins partiellement la matière organique et de libérer ainsi le métal ou les métaux contenu dans la plante.

La déshydratation et la destruction au moins partielle de la matière organique peut également être par déshydratation à l'étuve à température inférieure, de 50°C à 150°C, en particulier 100°C mais conduit à une composition dont la teneur en métal est différente (exemple 1).

Le terme métal doit être interprété dans un sens large et désigne des métaux tels que le zinc, le cuivre, le nickel, le fer, le chrome, le manganèse, le cobalt, l'aluminium, le plomb, le cadmium, l'arsenic, le thallium ou le palladium mais aussi des alcalino terreux tels que le magnésium ou le calcium ou des alcalins tels que le sodium ou le potassium.

Lesdits métaux sont principalement sous forme cationique.

L'expression « sous la forme M(II) » signifie que le métal est à un nombre d'oxydation égal à 2.

Cependant, la composition peut également contenir un ou plusieurs métaux sous une autre forme, c'est-à-dire avec un nombre d'oxydation différent, en particulier un nombre d'oxydation égal à 3 ou 1.

Dans la suite de la description, la plante ou les parties de plantes peuvent également être désignées matière végétale ou biomasse et ont la même signification.

Elle peut cependant également désigner les parties souterraines de la plante telles que les racines.

Par l'expression « catalyseur métallique », il faut comprendre un composé, comprenant au moins un métal sous la forme M(II), associé à un contre ion et qui, après mise en oeuvre dans une réaction de synthèse organique, va être récupéré sous la même forme que lorsqu'il a été mis en réaction et va donc pouvoir être recyclé pour la même réaction de synthèse organique ou pour une réaction de synthèse organique différente.

Le catalyseur peut également contenir des métaux à un degré d'oxydation différent.

L'expression « provenant de ladite plante » signifie que le métal ou les métaux présents dans la composition de l'invention sont originaires de la plante avant calcination et qu'il n'y a pas eu de rajout de métal provenant d'une autre

origine que ladite plante après la calcination, le traitement acide ou la filtration.

Les métaux tels que le zinc, le cuivre, le nickel, l'aluminium, le cobalt, le plomb, le chrome, le manganèse, l'arsenic ou le thallium ont été accumulées par la plante lors de sa croissance dans un sol contenant lesdites espèces.

Par contre, d'autres espèces cationiques telles que le $Mg^{2+}$, le $Ca^{2+}$, $Fe^{3+}$, $Na^{+}$ et $K^{+}$ n'ont donc pas été accumulées par la dite plante mais sont présentes physiologiquement dans ladite plante et proviennent par conséquent de celle-ci.

Concernant $Fe^{3+}$, le sol peut également contenir des concentrations importantes de cet ion métallique qui pollue la masse foliaire et provient donc également de la plante.

L'expression « dépourvue de chlorophylle » signifie que la composition ne contient plus de chlorophylle ou n'en contient que des résidus ou des traces en raison des différents traitements effectués lors de la préparation de la composition et notamment, la filtration après traitement acide.

Le traitement acide effectué après calcination permet de détruire totalement la matière organique présente dans la plante à l'origine.

La filtration permet d'éliminer les résidus de matière organique et notamment la chlorophylle ou les résidus de chlorophylle qui pourraient subsister après traitement acide.

Par l'expression « mise en oeuvre d'une réaction de synthèse organique faisant intervenir celui-ci », il faut comprendre la transformation d'un produit X en produit Y à l'aide du catalyseur et éventuellement d'un ou plusieurs autres produits.

Le métal est préférentiellement le zinc (Zn) le nickel (Ni) ou le cuivre (Cu) mais la composition peut également comprendre le cadmium (Cd), le plomb (Pb), l'arsenic (As), le cobalt (Co) ou le chrome (Cr), le manganèse (Mn) ou le thallium (Tl), le fer (Fe), le calcium (Ca), le magnésium (Mg), le sodium (Na(I)), le potassium (K(I)) ou l'aluminium(III).

L'un des avantages de l'invention est donc d'éliminer les métaux lourds présents dans les sites pollués et valoriser la biomasse contenant lesdits métaux lourds tout en fournissant une source de métaux pour des réactions de synthèse organique, évitant l'emploi de procédé énergétivores et consommateurs de solvants organiques nocifs pour l'environnement ainsi que le rejet d'effluents pollués.

Un autre avantage est la possibilité d'utiliser la composition contenant le catalyseur pour des réactions en milieu industriel.

Dans un mode de réalisation avantageux, la présente invention concerne l'utilisation d'une plante calcinée ou d'une partie de plante calcinée ayant accumulé au moins un métal sous forme M(II) et au moins un métal sous forme M(III), ledit métal sous forme M(II) étant choisi notamment parmi le zinc (Zn), le nickel (Ni) ou le cuivre (Cu), pour la préparation d'une composition contenant au moins un catalyseur métallique dont le métal est l'un des susdits métaux sous forme M(II) provenant de ladite plante, ladite composition étant dépourvue de chlorophylle, et permettant la mise en oeuvre de réactions de synthèse organique faisant intervenir ledit catalyseur.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu) telle que définie ci-dessus, dans laquelle ladite composition est dépourvue de charbon activé.

L'expression « dépourvue de charbon activé » signifie que la composition ne contient pas de charbon présentant une grande surface spécifique qui lui confère un fort pouvoir adsorbant.

Pour le charbon actif, la surface spécifique est de 500 à 2500 $m^2/g$.

Dans la suite de la description, l'expression « charbon actif » peut également être employée et a la même signification que l'expression « charbon activé ».

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu) telle que définie ci-dessus, dans laquelle ladite composition comprend moins d'environ 2%, notamment moins d'environ 0,2% en poids de C, en particulier environ 0,14%.

La calcination de ladite plante conduit non seulement à la destruction de la matière organique mais aussi à la transformation du carbone ainsi formé en $CO_2$ qui va être donc être presque complètement éliminé de la composition.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu) telle que définie ci-dessus, dans laquelle le traitement acide est effectué par de l'acide chlorhydrique, en particulier HCl gazeux, HCl 1N ou HCl 12N.

Dans un mode de réalisation avantageux, l'invention concerne l'utilisation d'une plante calcinée ou d'une partie de plante calcinée telle que définie ci-dessus, dans laquelle ledit au moins un métal sous forme M(II) est choisi parmi le zinc (Zn), le nickel (Ni), le manganèse (Mn), le plomb (Pb), le cadmium (Cd), le calcium (Ca), le magnésium (Mg) ou le cuivre (Cu), pour la préparation d'une composition contenant au moins un catalyseur métallique actif, sous forme M(II) provenant de ladite plante, ladite composition ayant été préalablement filtrée, après traitement acide, pour éliminer la chlorophylle, permettant ainsi la mise en oeuvre de réactions de synthèse organique faisant intervenir ledit catalyseur.

Une plante est capable d'accumuler ou de contenir un ou plusieurs métaux et par conséquent la composition

peut comprendre un métal choisi parmi : Zn, Ni, Mn, Na(I), K(I), Pb, Cd, Ca, Mg, Co, As ou Cu.

Elle peut comprendre également du fer qui est à l'origine présent sous forme M(III) mais qui après réduction, est présent uniquement sous forme M(II).

Elle peut comprendre de plus de l'aluminium qui est présent sous forme M(III).

Dans toute la description, lorsque le degré d'oxydation du métal M(I), M(II) ou M(III) n'est pas précisé, il s'agit de la forme M(II).

La composition peut comprendre deux métaux choisis parmi ceux cités ci-dessus.

La composition peut comprendre trois métaux choisis parmi ceux cités ci-dessus.

La composition peut comprendre quatre métaux choisis parmi ceux cités ci-dessus.

La composition peut comprendre cinq métaux choisis parmi ceux cités ci-dessus.

La composition peut comprendre six métaux choisis parmi ceux cités ci-dessus.

La composition peut comprendre sept métaux choisis parmi ceux cités ci-dessus.

La composition peut comprendre huit métaux choisis parmi ceux cités ci-dessus.

La composition peut comprendre neuf métaux choisis parmi ceux cités ci-dessus.

La composition peut comprendre dix métaux choisis parmi ceux cités ci-dessus.

La composition peut comprendre onze métaux parmi ceux cités ci-dessus.

La composition peut comprendre douze métaux parmi ceux cités ci-dessus.

La composition peut comprendre treize métaux parmi ceux cités ci-dessus.

La composition peut comprendre les quatorze métaux cités ci-dessus.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu) telle que définie ci-dessus, dans laquelle ladite plante est choisie parmi la famille des Brassicaceae, notamment les espèces du genre *Thlaspi* en particulier *T. caerulescens, T. goesingense, T. tatrense, T. rotundifolium, T. praecox,* les espèces du genre *Arabidopsis, en particulier Arabidopsis hallerii,* et du genre *Alyssum,* en particulier *A. bertolonii, A. serpyllifolium,* des Fabaceae, en particulier *Anthyllis vulneraria,* des Sapotaceae, notamment les espèces *Sebertia acuminata, Planchonella oxyedra,* des Convolvulaceae, notamment les espèces *Ipomea alpina, Planchonella oxyedra,* des Rubiaceae, notamment les espèces *Psychotria douarrei,* en particulier *P. costivenia, P. clementis, P. vanhermanii,* des *Cunoniaceae,* notamment les *Geissois,* des Scrophulariaceae, en particulier les espèces du genre *Bacopa,* notamment *Bacopa monnieri,* les algues, en particulier les algues rouges, notamment les rhodophytes, plus particulièrement *Rhodophyta bostrychia,* les algues vertes ou les algues brunes.

Toutes les plantes appartenant aux familles des Brassicaceae, Fabaceae, Sapotaceae, Convolvulaceae ou Rubiaceae ne sont pas capables de croître sur un sol contenant des métaux lourds et d'accumuler lesdits métaux lourds dans les parties aériennes.

Par conséquent, dans la famille des Brassiceae, les genres *Thlaspi, Arabidopsis* et *Alyssum* sont les genres préférés mais sans être limité à celles-ci.

Dans la famille des Fabaceae, *Anthyllis vulneraria* est préférée mais sans être également limité à celle-ci.

Dans la famille Sapotaceae, les espèces *Sebertia acuminata, Planchonella oxyedra* sont les espèces préférées mais sans être limité à celles-ci.

Dans la famille de Convolvulaceae, les espèces *Ipomea alpina, Planchonella oxyedra* sont les espèces préférées mais sans être limité à celles-ci.

Dans la famille des Rubiaceae, les espèces *Psychotria douarrei,* en particulier *P. costivenia, P. clementis, P. vanhermanii* sont les préférées mais sans être limité à celles-ci.

Dans la famille des Scrophulariaceae, l'espèce *Bacopa monnieri* est la préférée mais sans être limité à celle-ci.

Enfin, dans les algues, R*hodophyta bostrychia* est l'espèce préférée mais sans être limité à celle-ci.

Le tableau I ci-dessous présente les différents genres, sans être limité à ceux-ci, capables d'accumuler des métaux tels que le nickel, le zinc, le cobalt et le cuivre, le plomb, le chrome, le manganèse ou le thallium.

Chaque genre est bien évidemment capable d'accumuler le métal cité et éventuellement un ou plusieurs autres, notamment le cadmium ou l'aluminium(III).

**TABLEAU I**

| Compilation d'après: AJM Baker & R R Brooks 1989. Terrestrial higher plants which hyperaccumulate metallic elements-A review of their distribution, ecology and phytochemistry. Biorecovery, 1, 81-126; Brooks, R R (editor). 1998. Plants that hyperaccumulate heavy metals. Cabi Publishing.Wallingford. U.K. | | | | |
|---|---|---|---|---|
| **NICKEL** | **ZINC** | **COBALT & CUIVRE** | **PLOMB** | **CHROME** |
| | | | | |

(suite)

| Compilation d'après: AJM Baker & R R Brooks 1989. Terrestrial higher plants which hyperaccumulate metallic elements-A review of their distribution, ecology and phytochemistry. Biorecovery, 1, 81-126; Brooks, R R (editor). 1998. Plants that hyperaccumulate heavy metals. Cabi Publishing.Wallingford. U.K. | | | | |
|---|---|---|---|---|
| **NICKEL** | **ZINC** | **COBALT & CUIVRE** | **PLOMB** | **CHROME** |
| Adiantum | Arabidopsis | Aeollanthus | Armeria | Dicoma |
| Agatea | Arenaria | Alectra | Polycarpaea | Sutera |
| Alyssum | Cardaminopsis | Anisopappus | Thlaspi | |
| Anthyllis | Haumaniastrum | Ascolepis | Noccaea | |
| Arenaria | Noccaea | Bacopa | Alyssum | |
| Argophyllum | Silene | Becium | | |
| Baloghia | Thlaspi | Buchnera | **MANGANESE** | |
| Berkheya | Viola | Bulbostylis | | |
| Blepharis | | Celosia | Alyxia | |
| Bornmuellera | | Commelina | Beaupreopsis | |
| Brackenridgea | | Crassula | Crotalaria | |
| Buxus | | Crotalaria | Grevilla | |
| Campanula | | Cyanotis | Eugenia | |
| Cardamine | | Eragrostis | Macadamia | |
| Casearia | | Faroa | Maytenus | |
| Chromolaena | | Gutenbergia | Virotia | |
| Chrysanthemum | | Haumaniastrum | | |
| Cleidion | | Hibiscus | **THALLIUM** | |
| Cnidoscolus | | Icomum | Iberis | |
| Cochlearia | | Ipomoea | | |
| Dicoma | | Lindernia | **ALUMINIUM** | |
| Dychapetalum | | Millotia | Melastoma | |
| Esterhazya | | Minuartia | Psychotria | |
| Euphorbia | | Monadenium | Symplocos | |
| Geissois | | Pandiaka | | |
| Heliotropium | | Rendlia | | |
| Homalium | | Silene | | |
| Hybanthus | | Sopubia | | |
| Indigofera | | Striga | | |
| Juncus | | Triumfetta | | |
| Justicia | | Vernonia | | |
| Lasiochlamys | | Vigna | | |
| Leucanthemopsis | | Xerophyta | | |
| Leucocroton | | | | |

(suite)

| Compilation d'après:<br>AJM Baker & R R Brooks 1989. Terrestrial higher plants which hyperaccumulate metallic elements-A review of their distribution, ecology and phytochemistry. Biorecovery, 1, 81-126; Brooks, R R (editor). 1998. Plants that hyperaccumulate heavy metals. Cabi Publishing.Wallingford. U.K. | | | | |
|---|---|---|---|---|
| **NICKEL** | **ZINC** | **COBALT & CUIVRE** | **PLOMB** | **CHROME** |
| Linaria | | | | |
| Lophostachys | | | | |
| Luzula | | | | |
| Merremia | | | | |
| Minuartia | | | | |
| Mitracarpus | | | | |
| Myristica | | | | |
| Noccaea | | | | |
| Oncotheca | | | | |
| Pancheria | | | | |
| Pearsonia | | | | |
| Peltaria | | | | |
| Phyllanthus | | | | |
| Planchonella | | | | |
| Psychotria | | | | |
| Rhus | | | | |
| Rinorea | | | | |
| Ruellia | | | | |
| Saxifraga | | | | |
| Sebertia | | | | |
| Senecio | | | | |
| Shorea | | | | |
| Solidago | | | | |
| Stachys | | | | |
| Stackhousia | | | | |
| Streptanthus | | | | |
| Thlaspi | | | | |
| Trichospermum | | | | |
| Trifolium | | | | |
| Trisetum | | | | |
| Turnera | | | | |
| Vellozia | | | | |
| Walsura | | | | |
| Xylosma | | | | |

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu) telle que définie ci-dessus, dans laquelle ladite plante appartient à la famille des Brassicacea, notamment *Thlaspi caerulescens* ou *Arabidopsis hallerii* et le métal accumulé par ladite plante est Zn.

Dans ce mode de réalisation, les plantes utilisées sont avantageusement *Thlaspi caerulescens* ou *Arabidopsis hallerii* qui accumulent toutes majoritairement du zinc, notamment sous forme de carboxylate (en particulier malate) de zinc, c'est-à-dire sous forme $Zn^{2+}$ (ou Zn(II)) ainsi que d'autres métaux en proportion plus faible.

Le catalyseur au zinc peut être obtenu par exemple selon l'exemple 1. Dans ce cas, le catalyseur obtenu est un acide de Lewis correspondant au dichlorure de zinc.

L'un des avantages de l'invention est donc de fournir un catalyseur ne nécessitant pas de purification poussée. En effet, la présence des autres sels métalliques (tel que par exemple du $CdCl_2$ ou autres) ne va pas gêner les réactions organiques mises en oeuvre et il n'est donc pas nécessaire comme dans les procédés classiques de procéder à une séparation totale et délicate des espèces métalliques présentes.

Dans un mode de réalisation avantageux, l'invention concerne l'utilisation d'une plante calcinée ou d'une partie de plante calcinée ayant accumulé au moins un métal sous forme M(II), en particulier le zinc, telle que définie ci-dessus, dans laquelle la concentration en Zn dans la plante est comprise d'environ 2700 mg/kg à environ 43700 mg/kg de poids sec de plante ou partie de plante, préférentiellement d'environ 2700 mg/kg à environ 13600 mg/kg de poids sec de plante ou partie de plante, plus préférentiellement d'environ 6000 mg/kg à environ 9000 mg/kg de poids sec de plante ou partie de plante, en particulier d'environ 7000 mg/kg à environ 8000 mg/kg de poids sec de plante ou partie de plante

En dessous de 2700 mg/kg, la proportion de zinc est trop faible pour pouvoir valoriser la biomasse contenant le zinc à des coûts raisonnables.

Au-delà de 43700 mg/kg, la proportion de zinc est trop élevée pour que la plante puisse stocker autant de métal.

Les concentrations présentes dans la plante peuvent changer largement selon la nature du substrat et la quantité de métaux dans le sol.

Pour être précis, les résultats obtenus sur 24 plantes de Thlaspi récoltées sur les sites miniers sont les suivants: la moyenne était de 7300 mg/kg avec un écart type de 3163 une valeur maximale de 13600 et une minimale de 2700.

En hydroponie, qui représente une culture de plantes réalisée sur substrat neutre et inerte (de type sable, pouzzolane, billes d'argile, solution nutritive, etc.), les valeurs peuvent être beaucoup plus élevées de l'ordre de 30000 mg/kg (jusqu'à 43710 mg/kg selon Brooks et Reeves).

Reeves, R. D. and Brooks, R. R., 1983 . European species of Thlaspi L. (Cruciferae) as indicators of nickel and zinc. J. Geochem. Explor. 18:275-283. Reeves, R. D. and Brooks, R. R., 1983 . Hyperaccumulation of lead and zinc by two metallophytes from a mining area in Central Europe. Environ. Pollut. 31:277-287.

Dans un mode de réalisation avantageux, l'invention concerne l'utilisation d'une plante calcinée ou d'une partie de plante calcinée ayant accumulé au moins un métal sous forme M(II), en particulier le zinc, pour la préparation d'une composition telle que définie ci-dessus, dans laquelle le zinc de ladite composition est à une concentration comprise d'environ 15000 à environ 800000 ppm, notamment d'environ 20000 à environ 80000 ppm, notamment d'environ 61000 à environ 67700 ppm.

Le catalyseur obtenu est donc un catalyseur au zinc, c'est-à-dire que le zinc est le seul composé métallique présent dans la composition ou le composé métallique majoritaire dans la composition.

Par ppm, utilisé également dans toute la suite de la description, il faut comprendre mg/kg.

Etant donné que pour une même plante, une variabilité saisonnière peut exister modifiant par conséquent la concentration en métaux dans la plante et par conséquent dans la composition et que de plus la détermination des valeurs de concentrations des métaux peut varier en fonction de la mesure, les valeurs des gammes de concentrations sont données dans toute la description avec une marge d'erreur de plus ou moins 8%, préférentiellement de plus ou moins 7%, en particulier une erreur standard de plus ou moins 5%.

Dans un mode de réalisation avantageux, l'invention concerne l'utilisation d'une plante calcinée ou d'une partie de plante calcinée ayant accumulé au moins un métal sous forme M(II), en particulier le zinc, telle que définie ci-dessus, dans laquelle ladite composition comprend de plus au moins l'un des métaux suivants Mg, Al(III), Ca, Fe(III), Cu, Cd, Pb, aux concentrations définies ci-dessus.

Dans un mode de réalisation avantageux, l'invention concerne l'utilisation d'une plante calcinée ou d'une partie de plante calcinée ayant accumulé au moins un métal sous forme M(II), en particulier le zinc, telle que définie ci-dessus, dans laquelle le zinc de la composition est à une concentration comprise d'environ 15000 à environ 800000 ppm, notamment d'environ 20000 à environ 80000 ppm, notamment d'environ 61 000 à environ 67700 ppm, ladite composition comprenant de plus l'un ou plusieurs métaux de la liste suivante aux concentrations suivantes :

- Mg(II) : d'environ 2500 à environ 25000 ppm, notamment d'environ 4400 à environ 15 000 ppm, notamment d'environ 11 800 à environ 13100 ppm;
- Ca(II) : d'environ 20000 à environ 100000 ppm, notamment d'environ 73000 à environ 91 000 ppm;

- Fe(III) : d'environ 900 à environ 75000 ppm, notamment d'environ 3100 à environ 30000 ppm, notamment d'environ 8700 à environ 28000 ppm;
- Cu(II) : d'environ 30 à environ 400 ppm, notamment d'environ 55 à environ 170 ppm, notamment d'environ 99 à environ 170 ppm;
- Cd(II) : d'environ 700 à environ 10000 ppm, notamment d'environ 1800 à environ 5600 ppm, notamment d'environ 5300 à environ 5600 ppm;
- Pb(II) : d'environ 200 à environ 40000 ppm, notamment d'environ 4600 à environ 15000 ppm, notamment d'environ 13000 à environ 15000 ppm;
- Al(III) : d'environ 200 à environ 15000 ppm, notamment d'environ 2400 à environ 6000 ppm, notamment d'environ 1500 à environ 4700 ppm;

Les teneurs en métaux dépendent non seulement de la plante utilisée mais encore de l'endroit dans lequel ladite plante a été cultivée et notamment de la teneur en métal du sol.

C'est pourquoi les gammes de métaux accumulés dans la plante peuvent être très larges.

Dans un mode de réalisation avantageux, l'invention concerne l'utilisation d'une plante calcinée ou d'une partie de plante calcinée ayant accumulé au moins un métal sous forme M(II), en particulier le zinc, telle que définie ci-dessus, dans laquelle ladite composition comprend au moins les métaux suivants Mg, Al(III), Ca, Fe(III), Cu, Zn, Cd, Pb, aux concentrations définies ci-dessus.

Dans un mode de réalisation avantageux, l'invention concerne l'utilisation d'une plante calcinée ou d'une partie de plante calcinée ayant accumulé au moins un métal sous forme M(II), telle que définie ci-dessus, dans laquelle ladite plante est une Sapotaceae, en particulier *Sebertia acuminata,* une Rubiaceae, ou une Brassicaceae, en particulier *Thlaspi goesingense* ou *Thlaspi caerulescens,* et le métal accumulé par ladite plante est Ni.

Dans ce mode de réalisation, les plantes utilisées sont avantageusement *Sebertia acuminata, Thlaspi caerulescens* ou *Thlaspi goesingense* ainsi qu'une Rubiaceae qui accumulent toutes majoritairement du nickel, notamment sous forme de carboxylate de nickel, c'est-à-dire sous forme $Ni^{2+}$ ainsi que d'autres métaux en proportion plus faible.

Le catalyseur au nickel peut être obtenu par exemple selon l'exemple 5. Dans ce cas, le catalyseur obtenu est un acide de Lewis correspondant au chlorure de nickel.

Dans un mode de réalisation avantageux, l'invention concerne l'utilisation d'une plante calcinée ou d'une partie de plante calcinée ayant accumulé au moins un métal sous forme M(II), en particulier du Nickel, telle que définie ci-dessus, dans laquelle la concentration en Ni dans la plante est comprise d'environ 1000 mg/kg à environ 36000 mg/kg de poids sec de plante ou partie de plante, préférentiellement d'environ 2500 mg/kg à environ 25000 mg/kg de poids sec de plante ou partie de plante, plus préférentiellement d'environ 2500 mg/kg à environ 19900 mg/kg de poids sec de plante ou partie de plante, en particulier d'environ 15000 mg/kg à environ 18000 mg/kg de poids sec de plante ou partie de plante

En dessous de 1000 mg/kg, la proportion de nickel est trop faible pour pouvoir valoriser la biomasse contenant le nickel à des coûts raisonnables.

Au-delà de 36000 mg/kg, la proportion de nickel est trop élevée pour que la plante puisse stocker autant de métal.

Les concentrations présentes dans la plante peuvent changer largement selon la nature du substrat et la quantité de métaux dans le sol.

En hydroponie, qui représente une culture de plantes réalisée sur substrat neutre et inerte (de type sable, pouzzolane, billes d'argile, solution nutritive, etc.), les valeurs peuvent être beaucoup plus élevées de l'ordre de 36000 mg/kg.

Dans un mode de réalisation avantageux, l'invention concerne l'utilisation d'une plante calcinée ou d'une partie de plante calcinée ayant accumulé au moins un métal sous forme M(II), en particulier le nickel, pour la préparation d'une composition telle que définie ci-dessus, dans laquelle le nickel de ladite composition est à une concentration comprise d'environ 150000 à environ 700000 ppm, notamment d'environ 185000 à environ 300000 ppm, notamment d'environ 185000 à environ 270000 ppm.

Le catalyseur obtenu est donc un catalyseur au nickel, c'est-à-dire que le nickel est le seul composé métallique présent dans la composition ou le composé métallique majoritaire dans la composition.

Dans un mode de réalisation avantageux, l'invention concerne l'utilisation d'une plante calcinée ou d'une partie de plante calcinée ayant accumulé au moins un métal sous forme M(II), en particulier le nickel, telle que définie ci-dessus, dans laquelle le nickel de la composition est à une concentration comprise d'environ 150000 à environ 700000 ppm, notamment d'environ 185000 à environ 300000 ppm, notamment d'environ 185000 à environ 270000 ppm, ladite composition comprenant de plus l'un ou plusieurs métaux de la liste suivante aux concentrations suivantes :

- Mg(II) : d'environ 9000 à environ 100000 ppm, notamment d'environ 50000 à environ 90000 ppm, notamment d'environ 78000 à environ 87000 ppm;
- Ca(II) : d'environ 60000 à environ 120000 ppm, notamment d'environ 93000 à environ 106000 ppm;

- Zn(II) : d'environ 5000 à environ 8000 ppm, notamment d'environ 5700 à environ 7100 ppm;
- Fe(III) : d'environ 200 à environ 2000 ppm, notamment d'environ 260 à environ 1800 ppm;
- Cu(II) : d'environ 4000 à environ 5000 ppm, notamment d'environ 4500 à environ 4700 ppm;
- Cd(II) : d'environ 10 à environ 40 ppm, notamment d'environ 14 à environ 20 ppm;
- Pb(II) : d'environ 200 à environ 1500 ppm, notamment d'environ 300 à environ 1200 ppm;
- Al(III) : d'environ 600 à environ 2000 ppm, notamment d'environ 800 à environ 1700 ppm;
- Mn(II) : d'environ 100 à environ 1500 ppm, notamment d'environ 260 à environ 1200 ppm;

Dans un mode de réalisation avantageux, l'invention concerne l'utilisation d'une plante calcinée ou d'une partie de plante calcinée ayant accumulé au moins un métal sous forme M(II), en particulier le nickel, telle que définie ci-dessus, dans laquelle ladite composition comprend au moins les métaux suivants Mg, Al(III), Ca, Fe(III), Cu, Zn, Cd, Pb, Ni, Mn aux concentrations définies ci-dessus.

Dans un mode de réalisation avantageux, le catalyseur à base de $NiCl_2$ est utilisé pour la mise en oeuvre de réaction dans lesquelles un acide de Lewis tel que $NiCl_2$ est utilisé, telle qu'une réaction de substitution électrophile alkylante (voir exemple 11) ou acylante.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu), telle que définie ci-dessus, dans laquelle ladite plante est une Convolvulaceae, notamment *Ipomea alpina* ou *Bacopa monnieri* et le métal accumulé par ladite plante est Cu.

Dans ce mode de réalisation, la plante utilisée est avantageusement *Ipomea alpina* ou *Bacopa monnieri,* qui accumulent toutes majoritairement du cuivre, c'est-à-dire sous forme $Cu^{2+}$ ainsi que d'autres métaux en proportion plus faible.

Le catalyseur au cuivre peut être obtenu par exemple selon l'exemple 9. Dans ce cas, le catalyseur obtenu est un acide de Lewis correspondant au chlorure cuivrique.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu), en particulier du cuivre, telle que définie ci-dessus, dans laquelle la concentration en Cu dans la plante est comprise d'environ 1000 mg/kg à environ 13700 mg/kg de poids sec de plante ou partie de plante.

En dessous de 1000 mg/kg, la proportion de cuivre est trop faible pour pouvoir valoriser la biomasse contenant le cuivre à des coûts raisonnables.

Au-delà de 13700 mg/kg, la proportion de cuivre est trop élevée pour que la plante puisse stocker autant de métal.

Les concentrations présentes dans la plante peuvent changer largement selon la nature du substrat et la quantité de métaux dans le sol.

En hydroponie, qui représente une culture de plantes réalisée sur substrat neutre et inerte (de type sable, pouzzolane, billes d'argile, solution nutritive, etc.), les valeurs peuvent être beaucoup plus élevées de l'ordre de 36000 mg/kg.

Dans un mode de réalisation avantageux, l'invention concerne l'utilisation d'une plante calcinée ou d'une partie de plante calcinée ayant accumulé au moins un métal sous forme M(II), en particulier le cuivre, pour la préparation d'une composition telle que définie ci-dessus, dans laquelle le cuivre de ladite composition est à une concentration comprise d'environ 6000 à environ 60000 ppm, notamment d'environ 10000 à environ 30000 ppm.

Le catalyseur obtenu est donc un catalyseur au cuivre, c'est-à-dire que le cuivre est le seul composé métallique présent dans la composition ou le composé métallique majoritaire dans la composition.

Dans un mode de réalisation avantageux, l'invention concerne l'utilisation d'une plante calcinée ou d'une partie de plante calcinée ayant accumulé au moins un métal sous forme M(II), en particulier le cuivre, telle que définie ci-dessus, dans laquelle le cuivre de la composition est à une concentration comprise d'environ 6000 à environ 60000 ppm, notamment d'environ 10000 à environ 30000 ppm, ladite composition comprenant de plus l'un ou plusieurs métaux de la liste suivante aux concentrations suivantes :

- Mg(II) : d'environ 6000 à environ 10000 ppm, notamment d'environ 7000 à environ 9000 ppm;
- Ca(II) : d'environ 70000 à environ 150000 ppm, notamment d'environ 90000 à environ 140000 ppm;
- Zn(II) : d'environ 1000 à environ 4000 ppm, notamment d'environ 1500 à environ 3400 ppm;
- Fe(III) : d'environ 3000 à environ 8000 ppm, notamment d'environ 4100 à environ 5700 ppm;
- Cd(II) : d'environ 300 à environ 600 ppm, notamment d'environ 380 à environ 520 ppm;
- Pb(II) : d'environ 800 à environ 2000 ppm, notamment d'environ 1000 à environ 1500 ppm;
- Al(III) : d'environ 1800 à environ 6500 ppm, notamment d'environ 2100 à environ 5500 ppm;

Dans un mode de réalisation avantageux, l'invention concerne l'utilisation d'une plante calcinée ou d'une partie de plante calcinée ayant accumulé au moins un métal sous forme M(II), en particulier le cuivre, telle que définie ci-dessus, dans laquelle ladite composition comprend au moins les métaux suivants Mg, Al(III), Ca, Fe(III), Cu, Zn, Cd, Pb, Ni, aux concentrations définies ci-dessus.

Dans un mode de réalisation avantageux, l'invention concerne l'utilisation d'une plante calcinée ou d'une partie de plante calcinée ayant accumulé au moins un métal sous forme M(II), telle que définie ci-dessus, dans laquelle la composition après filtration est mise en oeuvre sans purification ultérieure dans des réactions de synthèse organique choisies parmi les halogénations notamment d'alcools, les réactions électrophiles en série aromatique, notamment les substitutions, la synthèse de 3,4-dihydropyrimidin-2(1*H*)-one (ou thione), les réactions de cycloaddition, les réactions de transestérification, la synthèse de révélateurs d'acides aminés ou d'oximes, et l'hydrolyse catalysée des fonctions organiques soufrées notamment les thiophosphates.

Dans ce mode de réalisation, le catalyseur contenant majoritairement du zinc, ou du cuivre ou du nickel est utilisé sans purification, c'est-à-dire tel qu'obtenu après traitement acide et filtration et permet d'effectuer plusieurs types de réactions organiques.

Par halogénation d'alcools, aussi dénommé réaction de Lucas, il faut comprendre la transformation d'alcools (R-OH), qu'ils soient primaire, secondaires ou tertiaires, en dérivé halogéné correspondant (R-Hal), notamment en R-Cl, catalysée par un catalyseur au zinc.

Par « substitution électrophile en série aromatique », il faut comprendre une réaction au cours de laquelle un atome, en règle générale d'hydrogène, fixé à un cycle aromatique est substitué par un groupement électrophile : $ArH + EX \rightarrow ArE + HX$, également catalysées par un catalyseur au zinc ou au nickel.

(voir par exemple l'exemple 11)

Comme indiqué plus haut, le catalyseur est recyclable plusieurs fois, en particulier au moins quatre fois, sans perte d'activité et à titre d'exemple, le catalyseur au zinc a été recyclé 4 fois dans les substitutions électrophiles aromatiques sans aucune perte d'activité.

Il est également possible d'effectuer des réactions d'additions électrophiles où $ZnCl_2$ catalyse la réaction du chlorure de p-methoxybenzyle avec des alcènes pour conduire aux produits d'addition 1:1 correspondants (Bäuml, E.,Tscheschlok, K. ; Pock, R. and Mayr, H., 1978. Synthesis of $\gamma$-lactones from alkenes employing p-methoxybenzyl chloride as +CH2---CO-2 equivalent, Tetrahedron Lett. 29 :6925-6926).

La synthèse de 3,4-dihydropyrimidin-2(1*H*)-one (ou thione), ou réaction de Biginelli, correspond à la réaction d'un aldéhyde aromatique avec une urée ou une thiourée et un acétoacétate d'alkyle. Elle peut être catalysée aussi bien par le catalyseur au zinc que le catalyseur au nickel.

Les réactions de cycloaddition, aussi dénommées réaction de Diels-Alder correspondent à l'addition d'un diène sur un diènophile et sont catalysées par un catalyseur au zinc ou au nickel.

Les réactions de transestérification correspondent au remplacement d'un ester d'alkyle, par exemple de méthyle, éthyle, propyle ... par un autre, par traitement de l'ester par un alcool différent de celui constituant l'ester. Elles sont catalysées par le catalyseur au zinc.

Le catalyseur à base de $CuCl_2$ est utilisé pour la mise en oeuvre de réaction dans lesquelles un acide de Lewis tel que $CuCl_2$ est utilisé, telle qu'une réaction de substitution électrophile alkylante (voir exemple 11).

La synthèse de révélateurs d'acides aminés ou d'oximes correspond à l'utilisation du catalyseur au cuivre pour faire révéler des composés chimiques tels que les acides aminés ou les oximes (voir par exemple l'exemple 10).

L'hydrolyse catalysée des thiophosphates, correspond en particulier à la détoxification d'un pesticide de la famille des organophosphorés dénommé parathion dont la toxicité végétale, animale et humaine est avérée.

Ladite hydrolyse est catalysée de préférence par le catalyseur au cuivre mais peut également être effectuée par le catalyseur au zinc.

Dans un mode de réalisation avantageux, l'invention concerne l'utilisation d'une plante calcinée ou d'une partie de plante calcinée ayant accumulé au moins un métal sous forme M(II) choisi notamment parmi le zinc (Zn), le nickel (Ni) ou le cuivre (Cu), telle que définie ci-dessus, dans laquelle la composition est associée à un support solide, en particulier du charbon activé, des argiles notamment la montmorillonite, de l'alumine, de la silice, de la baryte, des silicates, des aluminosilicates, des composites à base d'oxyde métalliques tels que la ferrite.

Pour des raisons de réactivité, il peut être intéressant d'associer ladite composition à du charbon activé qui présente une grande surface spécifique permettant un fort pouvoir absorbant du catalyseur et donc des vitesses de réactions supérieures à celles effectuées sans charbon activé.

Pour certaines réactions, notamment les substitutions électrophiles aromatiques, la dispersion sur un support solide, en particulier de la montmorillonite ou de la silice imprégnée d'oxydes ferriques est nécessaire à la réaction sinon on observe une dégradation des produits de la réaction.

Les supports tels que la silice imprégnée d'oxydes métalliques, notamment ferriques ou la montmorillonite possèdent une surface spécifique allant de 5 $m^2/g$ à 800 $m^2/g$ respectivement.

Selon un autre aspect, l'invention concerne un procédé de préparation d'une composition dépourvue de chlorophylle, telle que définie ci-dessus, contenant au moins un catalyseur métallique sous forme M(II) dont le métal est choisi notamment parmi Zn, Ni ou Cu, comprenant les étapes suivantes :

a. calcination d'une plante ou d'une partie de plante ayant accumulé au moins un métal sous forme M(II) choisi notamment parmi Zn, Ni ou Cu, pour obtenir une plante calcinée ou une partie de plante calcinée,
b. agitation de la dite plante calcinée ou partie de plante calcinée dans un acide, en particulier l'acide chlorhydrique ou de l'acide sulfurique, pour déstructurer la plante ou partie de plante calcinée et pour obtenir un mélange contenant la plante ou partie de plante calcinée et déstructurée et au moins un catalyseur métallique dont le métal est choisi notamment parmi Zn, Ni ou Cu,
c. concentration du susdit mélange contenant la plante calcinée ou partie de plante calcinée et déstructurée et au moins un catalyseur métallique pour obtenir un mélange concentré contenant une plante ou partie de plante calcinée et déstructurée et au moins un catalyseur métallique choisi notamment parmi Zn, Ni ou Cu en proportion supérieure à celle obtenue en b.,
d. filtration du susdit mélange concentré pour obtenir un filtrat et un précipité, ledit filtrat correspondant à une composition brute dépourvue de chlorophylle contenant au moins un catalyseur métallique dont le métal sous forme M(II) est choisi notamment parmi Zn, Ni ou Cu, et le pH dudit filtrat étant ajusté en fonction du métal, dans des conditions telles que le pH de la composition est ≤ 5 pour le Zn, environ égal à 7 pour le Ni et compris entre 2 et 7 pour le Cu.

La première étape a. de calcination est effectuée par chauffage élevé et permet d'éliminer l'eau présente et de détruire en grande partie la biomasse.
Elle peut également être effectuée par déshydratation par chauffage de la plante ou des parties de plantes, c'est-à-dire de la biomasse, puis broyage de la biomasse déshydratée.
Cette étape est déterminante pour l'obtention du catalyseur car elle conduit à la destruction plus ou moins importante de la matière végétale pour la faciliter sa dégradation totale par la suite en milieu acide.
La calcination permet d'obtenir une proportion finale de catalyseur plus importante que par déshydratation.
Le traitement acide de la deuxième étape b. permet de déstructurer la plante ou les parties de plantes, c'est-à-dire de détruire certaines membranes biologiques, notamment celles des vacuoles pour libérer les carboxylates métalliques, notamment de zinc et/ou de nickel et/ou de cuivre, et/ou d'autres métaux, pour former un chlorure métallique dans le cas de l'utilisation d'HCl ou un sulfate métallique dans le cas de l'utilisation d'acide sulfurique.
Le traitement permet également l'hydrolyse complète de la liaison ester entre la chaîne grasse et le noyau pyrrolique de la chlorophylle.
Dans les procédés classiques, la chlorophylle est éliminée par extraction à l'hexane. Lorsque cette méthode est employée dans l'invention à la place du traitement acide, le métal reste dans les vacuoles de la matière végétale et il ne peut être récupéré pour l'obtention du catalyseur.
Le milieu réactionnel contient donc un mélange de chlorures ou sulfates métalliques ainsi que d'autres composés de dégradation de la biomasse après deshydratation ou calcination et traitement acide ainsi que des produits de dégradation de la cellulose et de la chlorophylle notamment :
La concentration effectuée à l'étape c. permet d'augmenter la concentration du milieu en catalyseur métallique ainsi qu'en acide pour obtenir une efficacité optimale du catalyseur lors de la mise en oeuvre de la réaction organique. Le pH doit alors être acide pour éviter la formation et la précipitation des hydroxydes métalliques.
La dernière étape d. est également primordiale pour la mise en oeuvre du catalyseur.
En effet, elle permet d'éliminer totalement les résidus de chlorophylle qui reste sur le système de filtration, notamment un fritté, ce qui conduit à l'obtention d'un filtrat incolore contenant le catalyseur métallique qui ne contient donc plus de chlorophylle ou de résidus de chlorophylle.
Si l'étape d. est effectuée par centrifugation ou par lyophilisation, donc sans filtration, la mise en oeuvre de la réaction organique par la suite n'est pas possible car la chlorophylle ou les résidus de chlorophylle empêchent fortement la réaction et conduisent à un milieu fortement coloré.
Ainsi l'exemple 12 montre que la réaction sur un alcool secondaire effectuée avec une composition contenant un catalyseur au zinc, obtenue sans filtration, ne conduit pas au dérivé halogéné désiré (traces seulement après 5 heures de réaction), contrairement à la composition de l'exemple 3, obtenue avec filtration, qui conduit au dérivé halogéné avec un rendement de 40% après 3 heures de réactions. La filtration permet l'obtention de réactions organiques avec un rendement au moins égal à 18% avec un traitement par HCl 1N et deshydratation, en particulier 47 à 94% avec un traitement par HCl 12N et calcination.
Dans un mode de réalisation avantageux, le procédé ci-dessus défini permet l'obtention de réactions organiques avec un rendement au moins supérieur à 18%.
Dans un mode de réalisation avantageux, le procédé ci-dessus défini permet l'obtention de réactions organiques, sauf dans le cas de l'alcool primaire : hexanol-1, avec un rendement au moins supérieur à 35%

Le pH doit être contrôlé après filtration à une valeur qui est fonction du métal utilisé pour conduire à une composition ayant par exemple un pH ≤ 5 pour le Zn, environ égal à 7 pour le Ni et compris entre 2 et 7 pour le Cu pour que la réaction organique puisse être mise en oeuvre par la suite. En effet, le catalyseur métallique à ce pH reste en solution et ne précipite pas.

Au cas où le pH est supérieur à 5 dans le cas du zinc ou pour les métaux nécessitant un pH acide, il doit être corrigé à une valeur inférieure ou égale à 2 par ajout d'acide, notamment d'HCl dilué ou concentré, c'est-à-dire d'HCl 0,1N, ou 1N à 12N, ou encore d'HCl gazeux par barbotage.

La composition obtenue contient donc au moins un catalyseur métallique ainsi que des composés de dégradation de la matière première végétale tels que des produits de dégradation totale ou partielle de la cellulose, tels que le cellobiose qui provient de la dépolymérisation de la cellulose et qui lui même peut être dégradé totalement ou partiellement en glucose qui peut être lui-même dégradé totalement ou partiellement en produits tels que le 5-hydroxyméthylfurfural ou l'acide formique.

Dans un mode de réalisation avantageux, l'invention concerne un procédé de préparation d'une composition telle que définie ci-dessus, dans lequel :

l'étape a. est effectuée à une température comprise d'environ 200 °C à environ 800°C, en particulier 300°C durant environ 1h à 3 h, en particulier 2h, puis refroidissement à 25°C,
l'étape b. est effectuée avec de l'acide chlorhydrique gazeux, de l'acide chlorhydrique aqueux dilué ou concentré, en particulier concentré, durant environ 30 minutes à environ 2h, en particulier 1h,
l'étape c. est effectuée :

- par évaporation partielle ou,
- par sonication durant environ 1 à environ 3 heures et addition d'acide chlorhydrique dilué, concentré.

La calcination de l'étape a. doit être faite à une température suffisante pour calciner, c'est-à-dire pour obtenir une combustion totale de la biomasse mais pas trop haute non plus car le procédé devient difficilement applicable en milieu industriel.

En dessous de 200°C, la température ne permet pas une combustion totale.

Au dessus de 800°C, la température est trop importante pour être facilement utilisée en milieu industriel.

L'acide utilisé est préférentiellement de l'acide chlorhydrique gazeux, ou aqueux et peut être dilué ou concentré, c'est-à-dire HCl 0,1N, ou 1N à 12N. Cependant les meilleurs résultats pour la mise en oeuvre de la réaction organique par la suite sont obtenus avec de l'HCl concentré soit 12N.

La sonication permet de détruire de manière plus importante la chlorophylle et provoque un échauffement ce qui conduit à une concentration du milieu. Il est cependant nécessaire d'ajouter de l'acide chlorhydrique concentré (12N) pour contrôler le pH.

La sonication conduit donc à l'obtention d'un catalyseur métallique avec un rendement supérieur au procédé sans sonication.

En dessous de 1h, l'échauffement provoqué n'est pas assez important pour concentrer suffisamment, au delà de trois heures la concentration devient trop importante.

Au cas où la sonication n'est pas effectuée, une évaporation partielle est nécessaire pour augmenter la concentration en acide.

La composition contient donc au moins un catalyseur métallique tel que du dichlorure de zinc et/ou du dichlorure de nickel et/ou du chlorure cuivrique en proportion majoritaire et/ou un chlorure métallique constitué d'autre métaux tels que le plomb, le cadmium, l'arsenic, le cobalt, le chrome, le manganèse ou le thallium en fonction de la proportion en métaux présente dans la plante avant calcination, ainsi que les composés de dégradation de la matière première végétale après les différentes étapes du procédé.

Dans un mode de réalisation avantageux, la composition obtenue par le procédé ci-dessus après traitement acide est dépourvue de charbon activé.

Dans un mode de réalisation avantageux, la composition obtenue par le procédé ci-dessus après traitement acide comprend moins d'environ 2%, notamment moins d'environ 0,2% en poids de C, en particulier environ 0,14%.

Dans un mode de réalisation avantageux, l'invention concerne un procédé de préparation d'une composition telle que définie ci-dessus, dans lequel ladite plante appartient à la famille des Brassicacea, notamment *Thlaspi caerulescens* ou *Arabidopsis hallerii,* ledit acide est HCl1N et le métal de la dite composition est le Zn et comprend éventuellement au moins un métal choisi parmi Mg, Ca, Fe(III), Al(III), Cu, Cd, Pb, Na, Mn, Ni.

Dans un mode de réalisation avantageux, l'invention concerne un procédé de préparation d'une composition telle que définie ci-dessus, dans lequel ladite plante appartient à la famille des Brassicacea, notamment *Thlaspi caerulescens* ou *Arabidopsis hallerii,* ledit acide est HCl 12N et le métal de la dite composition est le Zn, et ladite composition comprend éventuellement au moins un métal choisi parmi : Mg, Ca, Fe(III), Al(III), Cu, Cd, Pb.

Dans un mode de réalisation avantageux, le zinc de la composition est à une concentration comprise d'environ 15000 à environ 800000 ppm, notamment d'environ 20000 à environ 80000 ppm, notamment d'environ 61 000 à environ 67700 ppm, ladite composition comprenant de plus l'un ou plusieurs métaux de la liste suivante aux concentrations suivantes :

- Mg(II): d'environ 2500 à environ 25000 ppm, notamment d'environ 4400 à environ 15 000 ppm, notamment d'environ 11 800 à environ 13100 ppm;
- Ca(II): d'environ 20000 à environ 100000 ppm, notamment d'environ 73000 à environ 91 000 ppm;
- Fe(III): d'environ 900 à environ 75000 ppm, notamment d'environ 3100 à environ 30000 ppm, notamment d'environ 8700 à environ 28000 ppm;
- Cu(II): d'environ 30 à environ 400 ppm, notamment d'environ 55 à environ 170 ppm, notamment d'environ 99 à environ 170 ppm;
- Cd(II): d'environ 700 à environ 10000 ppm, notamment d'environ 1800 à environ 5600 ppm, notamment d'environ 5300 à environ 5600 ppm;
- Pb(II): d'environ 200 à environ 40000 ppm, notamment d'environ 4600 à environ 15000 ppm, notamment d'environ 13000 à environ 15000 ppm;
- Al(III): d'environ 200 à environ 15000 ppm, notamment d'environ 2400 à environ 6000 ppm, notamment d'environ 1500 à environ 4700 ppm.

Dans un mode de réalisation avantageux, l'invention concerne un procédé de préparation d'une composition telle que définie ci-dessus, dans lequel ladite plante est une Sapotaceae, en particulier *Sebertia acuminata,* une Rubiaceae, en particulier *Psychotria douarrei,* ou une Brassicaceae, en particulier *Thlaspi goesingense* ou *Thlaspi caerulescens,* ledit acide est HCl 12N et le métal de la dite composition est le Ni, et ladite composition comprend éventuellement au moins un métal choisi parmi : Mg, Al(III), Ca, Fe(III), Cu, Zn, Cd, Pb, Mn.

Dans un mode de réalisation avantageux, l'invention concerne un procédé de préparation d'une composition telle que définie ci-dessus, dans lequel ladite plante est une Convolvulaceae, notamment *Ipomea alpina* ou une Brassicaceae, en particulier *Thlaspi caerulescens,* ou une Scrophulariaceae, en particulier *Bacopa monnieri,* ledit acide est HCl 12N et le métal de la dite composition est le Cu, et ladite composition comprend éventuellement au moins un métal choisi parmi: Mg, Al(III), Ca, Fe(III), Zn, Cd, Pb, Ni.

Dans un mode de réalisation avantageux, l'invention concerne un procédé de préparation d'une composition telle que définie ci-dessus, dans lequel l'eau de la composition obtenue dans l'étape d. est évaporée totalement pour obtenir une composition déshydratée contenant ledit catalyseur.

Pour la mise en oeuvre de certaines réactions organiques, il est nécessaire de disposer d'un catalyseur ne contenant pas ou très peu d'eau.

Par conséquent, l'évaporation permet l'obtention d'un milieu déshydraté où seul le catalyseur fortement hygroscopique peut rester associé à un nombre limité de molécules d'eau.

Selon un autre aspect, la présente invention concerne un procédé de mise en oeuvre d'une réaction de synthèse organique comprenant une étape de mise en contact d'une composition dépourvue de chlorophylle contenant au moins un catalyseur métallique dont le métal sous forme M(II) est choisi notamment parmi Zn, Ni ou Cu, telle que définie ci-dessus, avec au moins un composé chimique susceptible de réagir avec ladite composition.

L'un des avantages de l'invention est de pouvoir utiliser directement la composition contenant le catalyseur obtenu ci-dessus, sous forme aqueuse acide ou sous forme déshydratée sans purification ultérieure, et de le mettre en présence d'un ou plusieurs réactifs chimiques pour conduire une réaction chimique.

Selon un autre aspect, la présente invention concerne un procédé de mise en oeuvre d'une réaction de synthèse organique, tel que défini ci-dessus, dans lequel ladite réaction de synthèse organique est choisie parmi les halogénations notamment d'alcools, les réactions électrophiles en série aromatique, notamment les substitutions ou additions, la synthèse de 3,4-dihydropyrimidin-2(1*H*)-one ou de 3,4-dihydropyrimidin-2(1*H*)-thione, les réactions de cycloaddition, et la synthèse de révélateurs d'acides aminés ou d'oximes.

Dans un mode de réalisation avantageux, la présente invention concerne un procédé de mise en oeuvre d'une réaction d'halogénations notamment d'alcools, tel que défini ci-dessus, comprenant les étapes suivantes :

a. mise en contact d'un alcool avec une composition contenant ledit catalyseur métallique, dont le métal sous forme M(II) est Zn et ayant un pH inférieur ou égale à 5, telle que définie ci-dessus, pour former un complexe alcool-catalyseur.

b. agitation dudit complexe à température ambiante ou à une température comprise d'environ 20 à 60°C, préférentiellement d'environ 20 à 50°C, en particulier d'environ 40°C durant environ 1 à environ 24h, préférentiellement environ 1 à environ 12h, plus préférentiellement d'environ environ 1 à environ 6h, en particulier environ 3h pour obtenir un mélange réactionnel contenant ledit dérivé halogéné,

c. extraction dudit mélange réactionnel par un solvant organique, en particulier de l'éther de pétrole, pour récupérer ledit dérivé halogéné.

Par complexe « alcool-catalyseur », il faut comprendre par exemple la formation d'un complexe de type acide-base de Lewis entre l'alcool et $ZnCl_2$ :

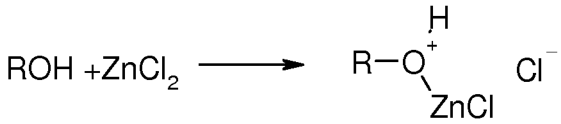

Ledit complexe est ensuite attaqué par l'ion chlorure qui par substitution nucléophile de type Sn2 conduit au dérivé halogéné par chauffage plus ou moins prononcé et durant un temps plus ou moins important en fonction de la réactivité de l'alcool :

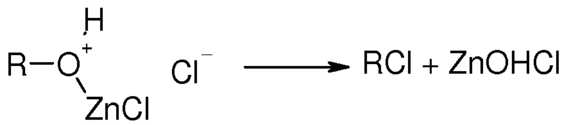

Le catalyseur est ensuite régénéré par le milieu acide pour reformer $ZnCl_2$ :

$$ZnOHCl + HCl \rightarrow ZnCl_2 + H_2O$$

L'alcool utilisé peut être un alcool primaire, secondaire ou tertiaire et l'exemple 3 présente plusieurs alcools sur lesquels la réaction a été effectuée.

L'exemple 4 présente une modélisation d'une réaction d'halogénation provoquée dans une espèce métallophyte.

Du malate de zinc a été préparé à partir d'acide malique commercial et mis en contact avec de l'HCl pour former le catalyseur $ZnCl_2$ qui a été mis en réaction avec le 4-méthyl-pentan-2-ol qui joue le rôle de solvant et de réactif.

L'alcool est alors halogéné (chloration) de la même manière qu'avec un métal provenant d'une plante qui accumule du zinc.

Dans un mode de réalisation avantageux, la présente invention concerne un procédé de mise en oeuvre d'une réaction d'halogénations notamment d'alcools, tel que défini ci-dessus, dans lequel le rapport molaire catalyseur/alcool de l'étape a. est compris d'environ 0,01 à environ 5, préférentiellement d'environ 0,1 à environ 5, plus préférentiellement d'environ 1 à environ 4, en particulier d'environ 2 à 4.

Le rapport molaire entre le catalyseur et l'alcool est fonction de l'alcool utilisé.

L'un des avantages de l'invention est de pouvoir utiliser le catalyseur en quantité catalytique, c'est-à-dire très inférieure à la stoechiométrie nécessitée par l'alcool, en proportion par exemple de 0,01% par rapport à l'alcool.

En dessous de cette limite, la réaction est trop lente pour pouvoir être effectuée.

Cependant, la réaction est plus rapide avec une proportion supérieure à la stoechiométrie et les valeurs en catalyseur avantageusement utilisées (en moles) sont entre 2 et 4 fois le nombre de moles d'alcool.

Au delà de 5, la proportion en catalyseur devient rédhibitoire pour le coût.

Dans un mode de réalisation avantageux, la présente invention concerne un procédé de mise en oeuvre d'une réaction de synthèse organique, tel que défini ci-dessus, dans lequel ladite réaction de synthèse organique est une réaction de substitution électrophile en série aromatique faisant intervenir deux réactifs **A** et **B.**

Un autre avantage de l'invention est de pouvoir effectuer d'autres réactions de synthèse organique que l'halogénation des alcools, et notamment des réactions de substitutions électrophiles telles que par exemple des réactions de Friedel et Craft telle que la réaction de l'exemple 11.

Dans un mode de réalisation avantageux, la présente invention concerne un procédé de mise en oeuvre d'une réaction de synthèse organique, en particulier une réaction de substitution électrophile, telle que définie ci-dessus, comprenant les étapes suivantes :

a. mise en contact des réactifs **A** et **B** avec une composition déshydratée telle que définie ci-dessus, et contenant ledit catalyseur métallique, dispersé sur un support solide, dont le métal est le Zn, le nickel ou l'aluminium, dans le toluène pour obtenir un complexe réactifs **A, B**-catalyseur,

b. agitation dudit complexe réactifs **A, B**-catalyseur à température ambiante ou à une température comprise d'environ 10 à 80°C, préférentiellement d'environ 15 à 40°C, en particulier d'environ 20°C durant environ 15 min à environ

15 h, préférentiellement environ 5 min à environ 2 h, en particulier environ 1 h, pour obtenir un produit de substitution électrophile,

c. filtration et évaporation pour récupérer ledit produit de substitution.

Si le catalyseur n'est pas dispersé sur un support solide minéral, la réaction conduit essentiellement à des produits de dégradation.

Le toluène de l'étape a. sert aussi bien de solvant que de réactif.

De la même manière que pour l'halogénation des alcools, un complexe est formé entre les réactifs et le catalyseur. Ledit complexe n'est cependant pas le même que celui obtenu pour les alcools.

La réaction se fait plus ou moins rapidement et nécessite plus ou moins de chauffage en fonction des réactifs utilisés. En dessous de 10°C, la réaction ne s'effectue pas. Au-delà de 80°C, il y a des risques de dégradation des réactifs.

Dans un mode de réalisation avantageux, la présente invention concerne un procédé de mise en oeuvre d'une réaction de synthèse organique, en particulier une réaction de substitution électrophile, telle que définie ci-dessus, dans lequel le rapport molaire catalyseur/**A** de l'étape a. est compris d'environ 0,01 à environ 5, préférentiellement d'environ 0,1 à environ 2, plus préférentiellement d'environ 1 à environ 4, en particulier d'environ 2 à 4, et le rapport molaire catalyseur/**B** de l'étape a. est compris d'environ 0,01 à environ 5, préférentiellement d'environ 0,1 à environ 5, plus préférentiellement d'environ 1 à environ 4, en particulier d'environ 2.

L'un des avantages de l'invention est de pouvoir utiliser le catalyseur en quantité catalytique, c'est-à-dire très inférieure à la stoechiométrie nécessitée par rapport à l'électrophile (le chlorure de benzyle dans l'exemple), en proportion par exemple de 0,01% par rapport aux réactifs A et B.En dessous de cette limite, la réaction est trop lente pour pouvoir être effectuée.

Cependant, la réaction est plus rapide avec une proportion supérieure telle que 0,1% de catalyseur.

Un second avantage est la possibilité de disperser le catalyseur sur un support solide minéral facilitant les opérations de séparation des produits et du catalyseur, puis du recyclage du catalyseur.

Dans un mode de réalisation avantageux, la présente invention concerne un procédé de mise en oeuvre d'une réaction de synthèse organique, tel que défini ci-dessus, dans lequel ladite réaction de synthèse organique est une réaction d'addition électrophile faisant intervenir deux réactifs C et D.

Dans un mode de réalisation avantageux, la présente invention concerne un procédé de mise en oeuvre d'une réaction de synthèse organique, en particulier une réaction d'addition électrophile telle que définie ci-dessus, comprenant les étapes suivantes :

a. mise en contact des réactifs C et D de la réaction avec une composition anhydre telle que définie ci-dessus, pour obtenir un complexe réactifs C, D-catalyseur

b. agitation dudit complexe C, D-catalyseur à température ambiante ou à une température comprise d'environ 20 à 100°C, pour obtenir un produit d'addition électrophile,

c. extraction par un solvant organique, pour récupérer ledit produit ledit produit d'addition électrophile.

Les solvants utilisables pour l'extraction sont bien connus de l'homme du métier.

Dans un mode de réalisation avantageux, la présente invention concerne un procédé de mise en oeuvre d'une réaction de synthèse organique, tel que défini ci-dessus, dans lequel le rapport molaire catalyseur/C est compris d'environ 0,01 à environ 5, préférentiellement d'environ 0,1 à environ 5, plus préférentiellement d'environ 1 à environ 4, en particulier d'environ 2 à 4, et le rapport molaire catalyseur/D étant compris d'environ 0,01 à environ 5, préférentiellement d'environ 0,1 à environ 5, plus préférentiellement d'environ 1 à environ 4, en particulier d'environ 2 à 4.

L'un des avantages de l'invention est de pouvoir utiliser le catalyseur en quantité catalytique, c'est-à-dire très inférieure à la stoechiométrie nécessitée par l'alcool, en proportion par exemple de 0,01% par rapport aux réactifs C et D.

En dessous de cette limite, la réaction est trop lente pour pouvoir être effectuée.

Cependant, la réaction est plus rapide avec une proportion supérieure à la stoechiométrie et les valeurs en catalyseur avantageusement utilisées (en moles) sont entre 2 et 4 fois le nombre de moles de réactif.

Au delà de 5, la proportion en catalyseur devient rédhibitoire pour le coût.

Dans un mode de réalisation avantageux, la présente invention concerne un procédé de mise en oeuvre d'une réaction de synthèse organique, dans lequel ladite réaction de synthèse organique est une réaction de synthèse de 3,4-dihydropyrimidin-2(1*H*)-one (ou thione).

Dans un mode de réalisation avantageux, ladite réaction de synthèse de 3,4-dihydropyrimidin-2(1*H*)-one (ou thione) comprend les étapes suivantes :

a. mise en contact d'un aldéhyde aromatique, d'une urée ou une thiourée et d'un acétoacétate d'alkyle avec une composition purifiée enrichie en zinc ou contenant du nickel et déshydratée, telle que définie ci-dessus, préalablement mélangée à un support solide minéral telle que de la silice, dans le toluène pour obtenir un complexe aldéhyde

aromatique-urée ou une thiourée -catalyseur,

b. agitation dudit complexe aldéhyde aromatique-urée ou une thiourée -catalyseur à une température comprise d'environ 80 à 120 °C, en particulier d'environ 110°C durant environ 1h min à environ 24 h, préférentiellement environ 5 min à environ 15 h, en particulier environ 10 h, pour obtenir une 3,4-dihydropyrimidin-2(1*H*)-one (ou thione),

c. filtration et évaporation pour récupérer ladite 3,4-dihydropyrimidin-2(1*H*)-one (ou thione).

Dans un mode de réalisation avantageux, la présente invention concerne un procédé de mise en oeuvre d'une réaction de synthèse organique, dans lequel ladite réaction de synthèse organique est une réaction de cycloaddition.

Dans un mode de réalisation avantageux, ladite réaction de cycloaddition comprend les étapes suivantes :

a. mise en contact d'un alcène en solution dans un solvant tel que le toluène avec une composition enrichie en zinc et fer et déshydratée, ou contenant du nickel ou de l'aluminium, telle que définie ci-dessus, en solution dans un solvant tel que le toluène, et agitation du milieu réactionnel pendant environ 1 min à 1h, en particulier 30 min à température ambiante, pour obtenir un complexe diénophile-catalyseur,

b. addition d'un diène et agitation pendant environ 5 min à 2h, en particulier 15 min à une température comprise entre - 70°C et 25°C pour obtenir un produit de cycloaddition,

c. hydrolyse, extraction liquide-liquide et évaporation pour récupérer ledit produit de cycloaddition.

Dans un mode de réalisation avantageux, la présente invention concerne un procédé de mise en oeuvre d'une réaction de synthèse organique, dans lequel ladite réaction de synthèse organique est une réaction d'hydrolyse catalysée des fonctions organiques soufrées notamment les thiophosphates.

Dans un mode de réalisation avantageux, ladite réaction d'hydrolyse catalysée des fonctions organiques soufrées comprend les étapes suivantes :

a. mise en contact d'un composé soufré à hydrolyser avec une composition enrichie en cuivre ou en Zinc et déshydratée, telle que définie ci-dessus, en solution dans un mélange de solvant tel que l'eau et l'éthanol, et agitation du milieu réactionnel pendant environ 24 à 48h, en particulier 30h à une température comprise de 20 à 80°C, en particulier 40°C, pour obtenir un composé soufré hydrolysé,

b. lavage en milieu basique, en particulier par de la soude et évaporation pour récupérer ledit composé hydrolysé.

Selon un autre aspect, la présente invention concerne une composition dépourvue de chlorophylle contenant au moins un catalyseur métallique dont le métal est choisi notamment parmi Zn, Ni ou Cu telle que définie ci-dessus, comprenant au moins un desdits métaux sous forme de chlorure, et des fragments cellulosiques de dégradation tels que du cellobiose et/ou du glucose, et/ou des produits de dégradation du glucose tels que le 5-hydroxyméthylfurfural et de l'acide formique et moins d'environ 2%, notamment moins d'environ 0,2% en poids de C, en particulier environ 0,14%.

La composition correspond donc à un ou plusieurs chlorures métalliques en fonction de la plante, du sol sur lequel elle a poussé et par conséquent des métaux qu'elle a pu absorber, dans le cas ou l'acide chlorhydrique a été utilisé pour le procédé de préparation de ladite composition.

Elle comprend également des produits de dégradation de la cellulose décrits ci-dessus qui cependant n'interfère pas pour le bon déroulement.

Dans un mode de réalisation avantageux, la présente invention concerne une composition contenant au moins un catalyseur métallique dont le métal est choisi notamment parmi Zn, Ni ou Cu telle que définie ci-dessus, en solution acidifiée, en particulier de l'acide chlorhydrique aqueux.

Dans ce mode de réalisation, la composition obtenue après la filtration ci-dessus définie, est obtenue en solution dans un acide, en particulier de l'acide chlorhydrique aqueux et peut être utilisée telle que sans purification ou traitement ultérieur pour la mise en oeuvre dans les réactions organiques.

Dans un mode de réalisation avantageux, la présente invention concerne une composition contenant au moins un catalyseur métallique dont le métal est choisi notamment parmi Zn, Ni ou Cu telle que définie ci-dessus, dépourvue de charbon activé.

Dans un mode de réalisation avantageux, la présente invention concerne une composition contenant au moins un catalyseur métallique dont le métal est choisi notamment parmi Zn, Ni ou Cu telle que définie ci-dessus, sous forme déshydratée.

Pour certaines réactions organiques à mette en oeuvre, il est nécessaire de disposer du catalyseur sans présence d'eau et par conséquent, la composition doit être déshydratée après obtention par le procédé de l'invention ou par un autre, avant utilisation, par évaporation ou par chauffage de manière à obtenir une composition ne comprenant pas ou très peu d'eau, où seul le catalyseur fortement hygroscopique peut rester associé à un nombre limité de molécules d'eau.

La préparation d'un catalyseur en milieu acide facilite sa déshydratation ultérieure: ainsi $NiCl_2$ est obtenu sans être associé à des molécules d'eau après une simple mise à l'étuve : la couleur jaune est le témoin de sa déshydratation totale.

Selon un autre aspect, la présente invention concerne une composition telle qu'obtenue par mise en oeuvre du procédé tel que défini ci-dessus.

## EXEMPLES

### Exemple 1:Préparation d'une composition contenant un catalyseur métallique dont le métal est Zn : obtention du catalyseur brut

30,03g de feuilles déshydratées et réduites en poudre de *Thlaspi caerulescens* issues du sol de la mine des Avnières sont dosées par la méthode Escarré (dosage au zincon) afin de mesurer le taux de zinc présent dans la matière sèche (sur les échantillons utilisés et calcinés : 420 mg ou 2 mmoles : *taux moyen et dépendant de l'endroit où les feuilles sont récoltées*). La matière sèche est ensuite placée dans 20 mL d'acide chlorhydrique 1N.

Remarque : la déshydratation est soit une calcination (environ 300°C pendant 2heures : on obtient alors des cendres), soit un chauffage de 100°C sous vide de 4 à 5 heures suivi d'un broyat au mortier). La masse de matière sèche est alors différente (plus de produits organiques dégradés et perdus par calcination, voir tableau II ci-dessous).

TABLEAU II

| Résultats en ppm (ICP-MS) | Mg | Al | Ca | Fe | Cu | Zn | Cd | Pb |
|---|---|---|---|---|---|---|---|---|
| déshydratation | 4394 | 2468 | 73827 | 3095 | 57 | 22501 | 1863 | 4653 |
| calcination | 11816 | 4726 | 90860 | 8738 | 99 | 61040 | 5498 | 12992 |

Les valeurs ci-dessus sont celles obtenues après traitement par HCl 1N et filtration.
Les métaux présents dans le tableau II sont sous forme M(II) sauf le fer qui est sous forme M(III).

Une alternative consiste à traiter la matière sèche par 20 ml d'HCl 12N.

Une analyse fine et détaillée de la composition des milieux a été effectuée par ICP-MS, méthode au zincon (pour le zinc) et polarographie impulsionnelle.

Les résultats sont tous concordants et répétés 3 fois (exprimés en ppm) ;

Cl a été dosé par la méthode de Mohr (formation du complexe rouge $Ag_2CrO_4$).

C et N ont été dosés par la méthode sèche CHN. Les valeurs moyennes sont résumées ci-après dans le tableau III :

TABLEAU III

| Catalyseur | Mg ppm | Ca ppm | Fe ppm | Cu ppm | Zn ppm | Cd ppm | Pb ppm | Na ppm | K ppm | P ppm | Mn ppm |
|---|---|---|---|---|---|---|---|---|---|---|---|
| Avec HCl 1N | 11816 | 90860 | 8738 | 99 | 61040 | 5498 | 12992 | 23000 | 28000 | 9500 | 360 |
| Avec HCl 12N | 13182 | 73827 | 27859 | 170 | 67744 | 5589 | 14946 | - | - | - | - |
| Catalyseur | Ni ppm | Co ppm | Cl ppm | C % | N % | | | | | | |
| Avec HCl 1N | 25 | - | ND | ND | ND | | | | | | |
| Avec HCl 12N | - | - | 3441 | 0,14 | 0,021 | | | | | | |
| ND : Non déterminé | | | | | | | | | | | |

Le traitement par HCl 12N modifie la composition, notamment il enrichit en zinc(II) et en fer(III) et diminue relativement la proportion de Ca.

La solution est agitée 1 heure, puis soniquée pendant 2 heures. Le milieu se concentre par échauffement du milieu réactionnel. 1 à 2 mL d'HCl 12N sont ajoutés pour permettre une agitation satisfaisante du milieu.

*Remarque : si la sonication n'est pas précisée, il faut prévoir une concentration du milieu réactionnel, puis l'ajout d'HCl 12N.*

La solution est filtrée sur un fritté de porosité 4. Le résidu solide est lavé par 2mL d' HCl 12N. Le filtrat doit être parfaitement limpide. Le pH est vérifié et éventuellement ajusté à une valeur inférieure à 2 si nécessaire par ajout d'HCl 12N. Une mesure rapide du zinc en solution par spectroscopie d'absorption atomique (spectromètre Spectra Varian AA 220FS) (*Thlaspi caerulescens, un indicateur de la pollution d'un sol ? Une réflexion partagée entre étudiants et chercheurs autour d'un problème environnemental* C. GRISON, J. ESCARRE, M.L. BERTHOMME, J. COUHET-GUICHOT, C. GRISON, F. HOSY, Actualité Chimique, 2010, 340, 27-32) permet de vérifier le taux de zinc récupéré (sous forme de $ZnCl_2$). Dans les conditions décrites, on récupère en moyenne 70% du zinc introduit initialement, soit ici 1,4 mmoles.

**Exemple 2 : Dosage du zinc dans les feuilles de plantes, après déshydratation, par spectrophotométrie UV-visible (Dosage au zincon, d'*après CEFE : centre d'écologie fonctionnelle et évolutive, Hélène Frérot et Bruno Buatois)***

**OBJET :**

Mesure de la concentration en zinc dans un échantillon végétal après dissolution du métal dans un acide, ajout d'un agent colorimétrique, et analyse par spectrophotométrie UV-visible de l'intensité de la coloration qui dépend de la quantité de zinc dans l'échantillon.

**Définitions :**

**Le zincon** = [acide [*alpha* -(hydroxy-2 sulfo-5 phénylazo) benzylidène] hydrazino-2 benzoïque, sel monosodique

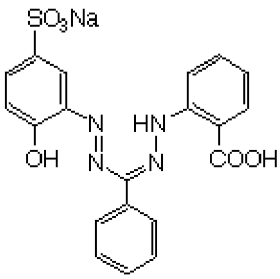

$C_{20}H_{15}N_4NaO_6S$ = 462.41 g.mol$^{-1}$
**Aspect** : poudre pourpre ou rougeâtre foncé
**Absorbance** : > 0.375 (autour de 490nm)
**Cendres sulfatées** : 15 - 25 %

Le zincon est un chélateur de métaux (Cu, Zn, Pb, Cd, Fe, Mn, Ni, Co, Al...). La chélation du zinc a lieu à pH 8.5-9.5. A ces pH, la solution aqueuse de zincon est de couleur orange, et vire au bleu en présence de zinc. A 606 nm, les valeurs d'absorbance d'une solution de zinc contenant du zincon donnent la concentration en zinc dans la solution.

**L'absorbance**

L'absorption de la lumière se traduit par un nombre de photons (une intensité lumineuse) plus faible à la sortie de l'échantillon qu'à l'entrée.

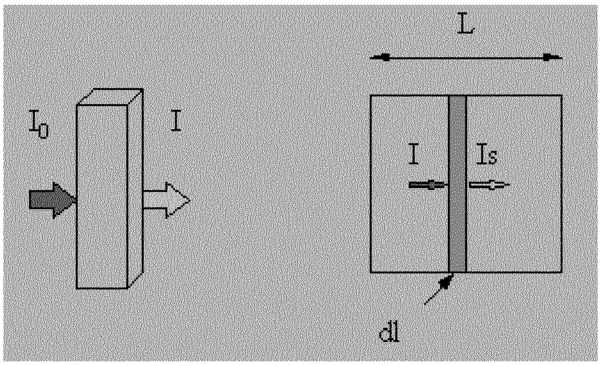


Is-I = -dI = k.c.I.dl qui donne dI/I = - k.c.dl qu'on intégre selon

$$\int_{I_0}^{I} \frac{dI}{I} = -k.c\int_{0}^{L} dl$$

qui donne Ln(I/Io)=-k.c.L.

On définit plutôt l'absorbance (A) selon A = log (I/Io) = -ε.c.L (loi de Beer-Lambert), où ε est le coefficient d'absorption molaire (en $M^{-1}.cm^{-1}$). On utilise aussi quelquefois la transmission T=I/Io.

Bien noter que 0<T<1 et 0<A<∞ et que l'absorbance est additive, tandis que la transmission ne l'est pas.

**Principe de la méthode :**

La méthode a été mise au point par Macnair & Smirnoff (Commun. Soil Sci. Plant Anal. 1999, 30, 1127-1136) pour *Arabidopsis halleri* et *Mimulus guttatus.* Elle a ensuite été appliquée pour *Thlaspi caerulescens.* Les mesures peuvent être moyennes (pour la plante entière : partie aérienne et/ou partie racinaire) ou ponctuelles (pour un morceau de feuille ou de racine). Les échantillons végétaux sont digérés par de l'acide sulfosalicylique, dans lequel le zinc va se dissoudre lentement. Une solution tampon à pH 9.6 permet de ramener le pH des échantillons à des valeurs compatibles avec la chélation du zinc par le zincon. La solution de zincon est ensuite ajoutée en quantité fixe. L'étalonnage est réalisé à l'aide de solutions étalon composées d'acide sulfosalicylique et de sulfate de zinc. La quantité de zincon doit rester supérieure à la quantité de zinc dans l'échantillon. De cette façon, le chélateur n'est pas saturé, tout le zinc contenu dans l'échantillon est susceptible d'être mesuré, et la valeur de l'absorbance se situe dans la gamme étalon. Une coloration bleue de l'échantillon après l'ajout de zincon indique sa saturation, donc la nécessité d'une dilution préalable aux mesures.

**Réactifs :**

**Solution d'acide sulfosalicylique à 2%** ($C_7H_6O_6S$, $2H_2O$ ; M=254,21 $g.mol^{-1}$ ; irritant pour les yeux et la peau ; en cas de contact avec les yeux, laver immédiatement et abondamment avec de l'eau et consulter un spécialiste)

- Dans un bécher de 250mL, peser 20g d'acide sulfosalicylique en poudre
- Ajouter de l'eau pure et mettre sous agitation magnétique jusqu'à dissolution complète
- Verser dans une fiole jaugée de IL (ou de 500mL) et compléter le volume à IL avec de l'eau pure
- Agiter à la main la solution finale

**Solution tampon pH=9.6**

- Etalonner le pH-mètre (voir protocole d'utilisation du pH-mètre)
- Dans un bécher de 250mL, peser 7.5g de chlorure de potassium (KCl ; 74,55 $g.mol^{-1}$)
- Dans un bécher de 250mL, peser 6.2g d'acide orthoborique ($H_3BO_3$ ; M=61,83 $g.mol^{-1}$)
- Ajouter de l'eau pure dans chaque bécher et mettre sous agitation magnétique jusqu'à dissolution complète
- Verser le contenu des deux béchers dans un seul bécher de 1L, et compléter à 800mL avec de l'eau pure

- Mettre sous agitation magnétique et placer l'électrode du pH-mètre dans la solution
- Préparer 100mL de solution d'hydroxyde de potassium à 2M, soit 11.22g dans 100mL d'eau pure (KOH ; M=56,11 g.mol$^{-1}$; R22-35 : nocif en cas d'ingestion, provoque de graves brûlures ; S26-36/37/39-45 : en cas de contact avec les yeux, laver immédiatement et abondamment avec de l'eau et consulter un spécialiste, porter un vêtement de protection approprié).
- A l'aide de la solution de KOH, amener doucement le pH à 9.6 (volume ajouté environ 50mL)
- Verser dans une fiole jaugée de IL (ou de 500mL) et compléter le volume à IL avec de l'eau pure
- Agiter à la main la solution finale

**Sulfate de zinc à 25mM** ($ZnSO_4$, $7H_2O$ ; M=287,54 g/mol; R36/38-50/53: irritant pour les yeux et la peau, très toxique pour les organismes aquatiques, peut entraîner des effets néfastes à long terme pour les organismes aquatiques; S22-25-60-61: ne pas respirer les poussières, éviter le contact avec les yeux, éliminer le produit et son récipient comme un produit dangereux, éviter le rejet dans l'environnement)

- Dans un bécher de 100mL, peser 0.719g de $ZnSO_4$, $7H_2O$
- Ajouter moins de 100mL d'acide sulfosalicylique à 2% et mettre sous agitation magnétique jusqu'à dissolution complète
- Verser le contenu du bécher dans une fiole jaugée de 100mL, et compléter le volume à 100mL avec de l'acide sulfosalicylique (ou bien peser 7.19g et mettre 10mL dans 100mL)

**Solution de zincon 0.03% à préparer juste avant utilisation**

- Dans un bécher, peser 0.03g de poudre de zincon (conservée sous vide dans un dessiccateur) pour 100mL de solution aqueuse. Ajouter le volume désiré d'eau pure, et mettre sous agitation magnétique dans un dessiccateur sous vide jusqu'à dissolution complète
- Agiter doucement à la main avant chaque utilisation (il peut rester de la poudre non dissoute)

**Appareillage :**

L'appareil utilisé est le spectrophotomètre Hολιο γ. Des cuves spéciales de contenance 1mL sont disposées sur un plateau tournant. Un faisceau lumineux de longueur d'onde donnée traverse les cuves sur leur face polie. Le plateau tournant comprend 7 emplacements. L'emplacement n°1 reçoit l'échantillon de référence servant à faire le zéro d'absorbance (0 nmol de zinc dans l'échantillon). Les 6 autres emplacements reçoivent les échantillons contenant le zinc à doser. Pour lire les valeurs d'absorbance, il suffit de faire tourner manuellement le plateau pour disposer successivement les cuves en face du faisceau lumineux.

**Etalonnage :**

**Solutions étalons (volumes de 1mL)**

- Préparer 6 tubes eppendorfs en inscrivant le nombre de moles dans 20 μL (volume d'un échantillon) de solution étalon
- Répartir dans les tubes les différents volumes de solution mère à 25mM, à l'aide de la pipette 20-200μL en prenant une pointe différente par tube
- Compléter les volumes à 1mL par de l'acide sulfosalicylique à 2% à l'aide de la pipette 100-1000μL

**Construction de la droite de calibrage**

1. Allumer le spectrophotomètre avec le bouton à l'arrière de l'appareil.
2. Attendre que l'appareil ait fait tous les tests.
3. Régler la longueur d'onde en appuyant sur le bouton correspondant à λm puis rentrer la longueur d'onde + ENTER.
4. Vérifier que l'on est bien en mode absorbance (dans MODE sélectionner ABS).
5. Dans chaque cuve de 1mL, mettre 780μL de solution tampon à l'aide de la pipette 100-100μL.

6. Ajouter 200μL de zincon à l'aide de la pipette 20-200μL; la couleur des mélanges varie de l'orange au bleu (bleu=saturation du chélateur).
7. Ajouter 20μL de solution étalon à l'aide de la pipette 20-200μL.
8. Homogénéiser le mélange dans chaque cuve à l'aide de la pipette 20-200μL et des pointes ayant servie aux prélèvements des solutions étalon.
9. Placer les cuves dans le plateau du spectro (attention à l'orientation par rapport au faisceau lumineux), telles que la cuve « 0 nmol » soit à l'emplacement n°1, « 10 nmol » à l'emplacement n°2, etc...
10. Appuyer sur « zéro base », l'appareil fait le zéro de l'absorbance pour la cuve n°1
11. Faire tourner le plateau d'un emplacement dans le sens inverse des aiguilles d'une montre, l'absorbance est alors indiquée pour la cuve n°2... jusqu'à la cuve n°7.
12. Vérifier que l'absorbance en fonction de la concentration de la solution étalon suit une relation linéaire (loi de Beer-Lambert), et noter la pente de la droite.
13. Eventuellement faire des réplicats ; vérifier le pH de 10mL de mélange pour spectrophotométrie, pour 0, 40 et 80 nmol.
14. La pente de cette droite intervient pour les calculs de la teneur en zinc des échantillons. La pente est le dénominateur.

**Echantillonnage :**

Préparation des échantillons pour une estimation de la concentration moyenne en zinc:

- Couper en petits fragments (matière fraîche) ou broyer à sec au mortier (matière sèche) la partie végétale à analyser (feuilles ou racines) pour chaque individu
- Mélanger les fragments et répartir dans plusieurs eppendorfs (au moins 4 par individu), à raison de 50 à 100mg de matière par eppendorf (remplissage à moitié environ) ; la masse des échantillons est à mesurer précisément en tarant chaque eppendorf avant la pesée d'un échantillon
- Si le matériel végétal est frais, faire un petit trou dans le bouchon des eppendorfs avant de les immerger 30 min dans l'azote liquide (laisser flotter dans un récipient en polystyrène fermé par un couvercle)
- Ajouter 1000 à 1500 μL d'acide sulfosalicylique à 2%: les plus faibles volumes sont utilisés lorsque la masse de tissus est faible et lorsque la concentration attendue en zinc est faible
- Laisser s'effectuer la digestion des tissus par l'acide pendant la nuit
- Dilution : prélever 100 microlitres de l'échantillon et le verser dans un autre eppendorf. Ajouter ensuite 300 microlitres d'acide sulfosalicylique pour obtenir une dilution x4. Il faudrait ajouter 700 pour une dilution par 8.

Préparation des échantillons pour une mesure ponctuelle:

- Couper en petits fragments (matière fraîche) ou broyer à sec au mortier (matière sèche) la partie végétale à analyser (feuilles ou racines) pour chaque individu
- Placer les fragments dans un eppendorf, à raison de 5 à 50mg de matière par eppendorf ; la masse des échantillons est à mesurer précisément en tarant chaque eppendorf avant la pesée d'un échantillon
- Si le matériel végétal est frais, faire un petit trou dans le bouchon des eppendorfs avant de les immerger 30 min dans l'azote liquide (laisser flotter dans un récipient en polystyrène)
- Ajouter 100 à 500 μL d'acide sulfosalicylique à 2%: les plus faibles volumes sont utilisés lorsque la masse de tissus est faible et lorsque la concentration attendue en zinc est faible
- Laisser s'effectuer la digestion des tissus par l'acide pendant la nuit
- Dilution : prélever 100 microlitres de l'échantillon et le verser dans un autre eppendorf. Ajouter ensuite 300 microlitres d'acide sulphosalycilique pour obtenir une dilution x4. Il faudrait ajouter 700 pour une dilution par 8.

**Mode opératoire :**

1. Allumer le spectrophotomètre
2. Dans chaque cuve de 1mL :

3. Mettre 780 μL de tampon à l'aide de la pipette 100-1000μL
4. Ajouter 200 μL de zincon **fraîchement préparé** à l'aide de la pipette 20-200μL
5. Prélever 20 μL d'échantillon à l'aide de la pipette 20-200μL ; si nécessaire pour prélever un liquide plus limpide, centrifuger l'eppendorf à 10000 trs/min pendant environ 8 min
6. Homogénéiser le mélange dans chaque cuve à l'aide de la pipette 20-200μL et des pointes ayant servie aux prélèvements des échantillons
7. Noter la couleur de l'échantillon ; si nécessaire (solution bleue = chélateur saturé) diluer l'échantillon en essayant d'en prélever le plus possible lors de la dilution
8. Mesurer l'absorbance à 606 nm par spectrophotométrie, et en déduire la teneur en zinc de l'échantillon (en nmol) grâce à la pente de la droite d'étalonnage

**Remarque importante :**

Le zincon craint l'oxydation, donc stocker la poudre à l'abri de l'air (dans une cloche à vide), protéger la solution prête à l'utilisation, et ne pas la conserver plus d'un jour.

**Exemple 3 : Réactions avec le catalyseur au Zinc de l'exemple 1**

**3.1 : Halogénation des alcools avec un catalyseur dont le métal est Zn**

**Exemple des alcools secondaires (procédure générale):**

De 0,5 à 2 mmoles, en particulier 1 mmole d'alcool (en fonction de l'alcool utilisé) est additionnée sur le mélange réactionnel de l'exemple 1 à 25°C.
Le temps moyen d'agitation est de 8 heures à 20°C. Le dérivé chloré peut-être isolé par ajout d'éther de pétrole, extraction, lavage avec une solution d'hydrogénocarbonate de sodium, séchage sur chlorure de calcium et élimination de l'éther de pétrole.
Un test de Beilstein et une analyse GC MS (VARIAN Chrompack CP 3800 Gas Chromatography / Varian MS Saturn 2000-Colonne optima 5 ; 30 m -0,25 μ - débit : 1 mL/min - Programme : 50°C : 2 min /100°C (montée : 5°C/min) ; 12 min /150°C) ; (montée : 20°C/min) ; 150°C : 16 min ; (montée : 50°C/min) ; 250°C : 17 min) confirment la formation du dérivé chloré.

**Extension du procédé aux alcools tertiaire et secondaire benzylique :**

Ces alcools ont été testés dans les mêmes conditions. La réaction est rapide (30 minutes).

**Extension du procédé aux alcools primaires :**

Le procédé est comparable, mais la réaction de chloration est plus difficile. Un chauffage poussé (reflux du milieu réactionnel) pendant 10 heures a été effectué.
Le tableau XI ci-dessous présente les mêmes réactions effectuées avec un catalyseur obtenu avec HCl 12N, utilisé brut (exemple 1) ainsi qu'une comparaison avec la réaction de Lucas effectuée selon les conditions standard bien connues de l'homme du métier :

TABLEAU XI

| | 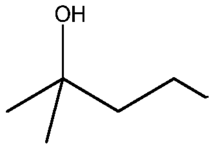 | 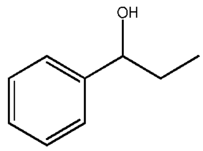 |
|---|---|---|
| PM | 102,10 | 136,09 |
| Nombre de moles | 0,7 à 1 mmole | 0,7 à 1 mmole |
| Conditions et catalyseur utilisé | Catalyseur brut 10 h à 25 °C | Catalyseur brut 10 h à 25 °C |
| Produits obtenus et rendement | 2-chloro-2-méthyl pentane : 47 % 3-chloro-3-méthyl pentane : 53 % | 1-chloro-1-phényl propane : 90 % |

(suite)

| | | |
|---|---|---|
| Comparaison avec la réaction standard de Lucas | 8 h à 25 °C 2-chloro-2-méthyl pentane : 47 % 3-chloro-3-méthyl pentane : 53 % | 2h à 25°C 1-chloro-1-phényl propane : 100% |

### 3.1: Substitution électrophile aromatique

Le catalyseur utilisé est brut (exemple 1 avec HCl 12N)
Il doit être dispersé sur montmorillonite ou de la silice imprégnée d'oxyde métallique
Il est recyclable au moins quatre fois.

### 3.1.1: Friedel et Craft alkylant

217 mg de catalyseur brut (exemple 1 avec HCl 12N) et sec sont dispersés et broyés au mortier avec 174 mg de Montmorillonite K10, puis chauffés à 110C dans un creuset.

Le dérivé halogéné (87 mmol) est ajouté à 20 équivalents du réactif aromatique. Le solide précédent est ajouté en une seule fois. Le mélange est agité selon le temps indiqué dans le tableau. On filtre, puis on concentre le milieu sous-vide. Le milieu est analysé en GC-MS et RMN $^1$H.

Les résultats sont présentés dans le tableau XII suivant :

TABLEAU XII

| Composé A | Composé B | conditions | Rendements régiomères | remarques |
|---|---|---|---|---|
| 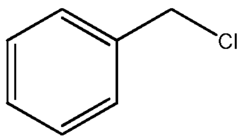 | 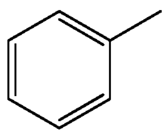 | 1h | 2,5% | Nombreux produits secondaires dont 2 adduits de Zn |
| 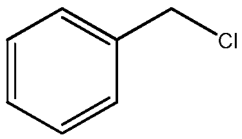 | 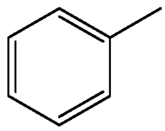 | 1h | 11% | |
| 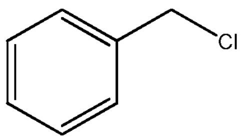 | 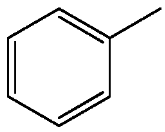 | 1h | 100%<br>Ortho : 18%<br>Para : 82% | |
| 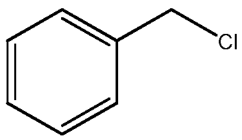 | 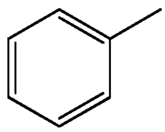 | 1h | 100%<br>Ortho : 18%<br>Para : 82% | Catalyseur recyclé |
| 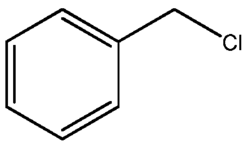 | 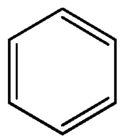 | 1h<br>9h<br>14h | 30%<br>52%<br>69% | |
| 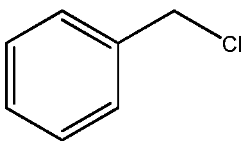 | 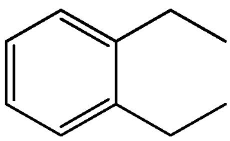 | 1h<br>14h | 0%<br>89%<br>Ortho : 31%<br>Para : 69% | |

(suite)

| Composé A | Composé B | conditions | Rendements régiomères | remarques |
|---|---|---|---|---|
| 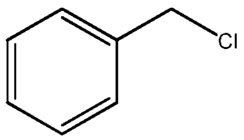 | 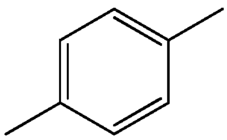 | 1h<br>14h | 52%<br>98% | |
| 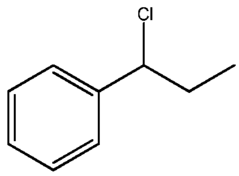<br>issu de la réaction de Lucas précédente (exemple 3, tableau XI) | 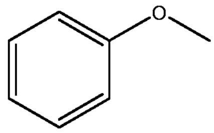 | 14h | 31%<br>Ortho : 30%<br>Para : 70% | |
| 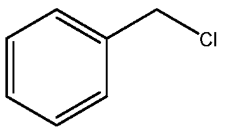 | 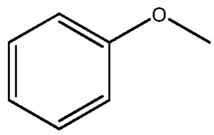 | 10 min | 100%<br>Ortho : 40%<br>Para : 60% | |

**3.1.2: Friedel et Craft acylant**

*Colorants :*

- phénolphtaléine

500mg d'anhydride phtalique, 500mg de phénol et 1g de catalyseur brut dérivé de Thlaspi (exemple 1, HCl 12N) déshydraté à 110°C quelques minutes sont placés dans un ballon monocol et chauffés à 80°C pendant 5 minutes.
Après refroidissement, le mélange réactionnel est dilué dans 5 mL d'un mélange eau/éthanol. 1mL de solution est prélevé puis additionné à une solution de soude 3M.
Dans le cas de la phénolphtaléine, la solution rosit immédiatement.
Après lavage à l'éther, la phénolphtaléine cristallise facilement.

- fluorescéine

500mg d'anhydride phtalique, 1 g de résorcinol et 2g de catalyseur brut dérivé de Thlaspi (exemple 1, HCl 12N) déshydraté à 110°C quelques minutes sont placés dans un ballon monocol et chauffés à 80°C pendant 5 minutes.
Après refroidissement, le mélange réactionnel est dilué dans 5 mL d'un mélange eau/éthanol.
1mL de solution est prélevé puis additionné à une solution de soude 3M.
Pour la fluorescéine, le mélange basique est versé dans une solution d'ammoniaque diluée. Une solution jaune vif fluorescente met en évidence la formation de la fluorescéine.

*ortho ou para éthyle acetophénone*

Placer 5 mL de toluène anhydre dans un tricol, puis introduire en une fois 4,5 g de catalyseur (exemple 1, HCl 12N). Ajouter goutte à goutte 0,7 mL d'anhydride acétique. Chauffer 30 minutes à 100°C. Laisser refroidir et verser le mélange réactionnel sur une solution glacée d'acide chlorhydrique concentré (10 mL). Verser dans une ampoule à décanter, puis séparer la phase organique. Laver celle-ci à l'eau, puis avec une solution aqueuse de chlorure d'ammonium jusqu'à pH=7.
Sécher la phase organique sur sulfate de sodium anhydre.
Les résultats sont présentés dans le tableau XIII :

Tableau XIII

| Composé A | Composé B | conditions | Rendements régiomères | remarques |
|---|---|---|---|---|
| 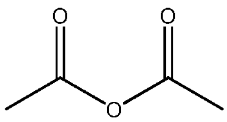 | 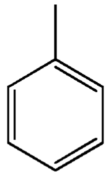 | 30 min | 95%<br>Ortho : 40%<br>Para : 60% | |
| 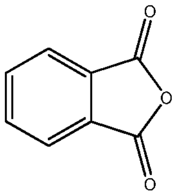 | 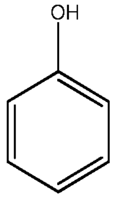 | 5 min | 90% | Extraction à l'éther F°C : 258-263<br>Test coloré à pH 9 |
| 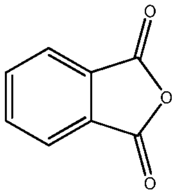 | 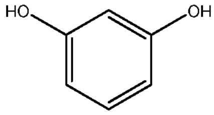 | 5 min | 90% | Lavages à EtOH 20°C-Mise en évidence de la fluorescence sous UV |

**3.2: Réactions de transestérification**

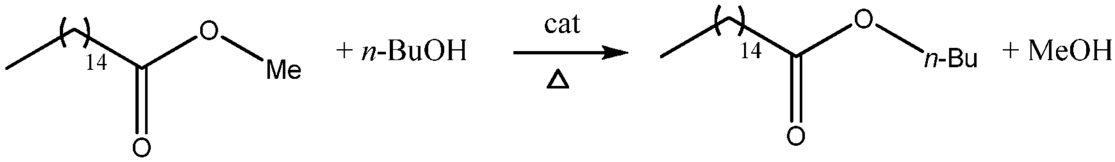


Une réaction modèle a été étudiée avec le palmitate de méthyle (270 mg, 1 mmol) et le butan-1-ol (5 mL). 100mg de catalyseur déshydraté issu de Thlaspi ont été ajoutés ; le mélange a été chauffé pendant 5h, puis 10h et analysé en GC-MS.
Si le catalyseur est utilisé brut (exemple 1, HCl 12N), la réaction a un degré d'avancement de 13 %.

**Exemple 4 : Modélisation d'une réaction d'halogénation provoquée dans une espèce métallophyte**

1) Préparation du malate de zinc, afin de générer au laboratoire l'espèce sous laquelle le zinc est présent dans *T. caerulecens ;*
2) Préparation de chlorure de zinc à partir du malate de zinc ;
3) Halogénation d'un alcool secondaire à l'aide du chlorure de zinc préparé précédemment.

La mise en oeuvre de ces transformations est effectuée comme suit :

1) le malate de zinc est préparé par action de zinc en poudre activé (activation préalable par $Me_3SiCl$) sur l'acide malique (Aldrich 088K0026). Ce dernier étant solide, une dissolution partielle et une homogénéisation du milieu sont effectuées à l'aide de 4-méthyl-pentan-2-ol. Cet alcool sert à la fois de solvant dans tout le procédé et d'alcool modèle dans la réaction d'halogénation ; le dégagement d'hydrogène, puis la dissolution totale du zinc permettent de suivre l'avancement de la réaction.

La réaction nécessite un chauffage à 50°C pour assurer la consommation totale de zinc, condition nécessaire pour que la suite réactionnelle soit significative (dans le cas contraire, le zinc réagit avec HCl à l'étape suivante pour former directement $ZnCl_2$).

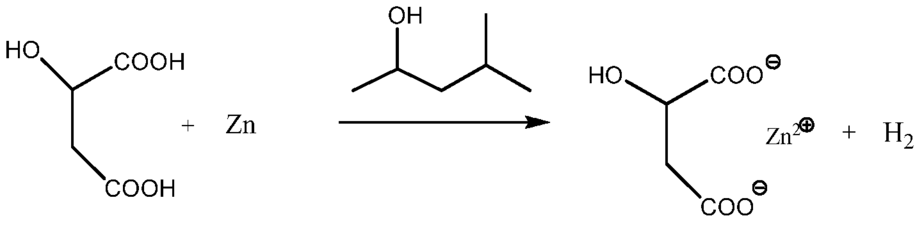

2) l'addition d'un excès d'acide chlorhydrique au malate de zinc permet par simple réaction acide-base de former le dichlorure de zinc et d'entraîner la préparation *in situ* du réactif de Lucas.

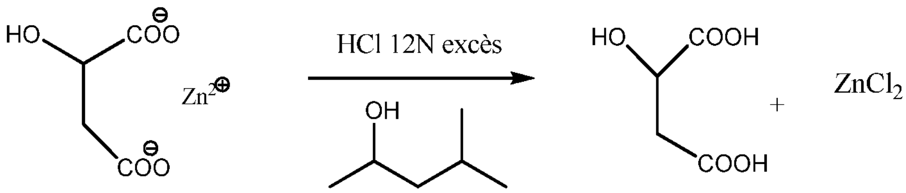


3) Le mélange $ZnCl_2$ / HCl étant formé en présence de 4-méthyl-pentan-2-ol, la réaction d'halogénation démarre dès addition d'HCl.

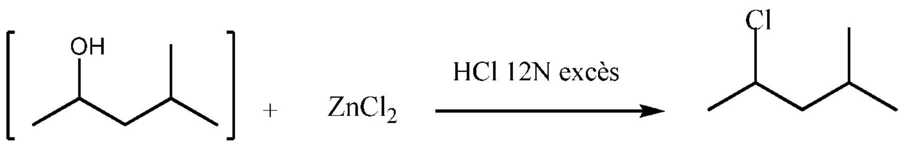


Après 15 minutes d'agitation à température ambiante, le mélange réactionnel est traité. Le taux de conversion évalué en GC MS est de 60%.

**Conclusion**

La séquence réactionnelle effectuée en milieu végétal est donc parfaitement modélisée dans des conditions classiques de synthèse.

**Partie expérimentale**

Dans un ballon monocol de 100 mL, muni d'un réfrigérant à eau, sont successivement introduits 2,534 g d'acide malique (0,0189 mol) sous forme solide, ainsi que 2,472 g de zinc métal (0,018 mol) sous forme de poudre ; le 4-méthyl-pentan-2-ol (7 mL) est ajouté pour disperser les solides et faciliter l'agitation du milieu réactionnel dans lequel l'acide malique est partiellement soluble.

Le mélange est porté à reflux pendant 4 heures à 50 °C, puis il est ramené à température ambiante sous agitation pendant 12 heures jusqu'à consommation totale du zinc métal.

L'acide chlorhydrique 12N (6 éq.) est ensuite ajouté au mélange afin de générer $ZnCl_2$.

Enfin, l'excès de 4-méthyl-pentan-2-ol réagit avec l'acide malique régénéré pour donner le 2-chloro-4-méthyl-pentane. 15 mL d'éther sont ajoutés pour extraire le dérivé chloré. Après décantation et séparation des phases aqueuses et organiques, la phase éthérée est lavée par deux fois 10 mL d'eau puis séchée sur sulfate de magnésium. La solution est filtrée puis concentrée. Le mélange brut est distillé (Eb= 131-134°C). 60% de 2-chloro-4-méthylpentane (1,285 g) sont isolés purs.

La solution est soumise au test de Beilstein afin de vérifier indirectement la présence de $ZnCl_2$. Le test est positif. La formation du dérivé chloré est facilement confirmée par spectrométrie de masse (m/z : 135 et 137).

**Exemple 5:Préparation d'une composition contenant un catalyseur métallique dont le métal est Ni**

Exemple 5.1 : Plante *Sebertia acuminata*

10 g de tiges et de petites branches de *Sebertia acuminata* sont calcinées. 4,5 à 5g de nickel sont ainsi obtenus. Les cendres sont placées dans un bécher contenant 30 mL d'HCl 12N. Le mélange est agité vigoureusement pendant 30 minutes à 50°C.

Le mélange est filtré, puis le filtrat est concentré et déshydraté à 110°C pour obtenir une composition déshydratée contenant un catalyseur au $NiCl_2$.

Exemple 5.2 : Plante *Psychotria douarrei*

*Calcination* : La calcination est réalisée selon le programme standard (300°C pendant 2h, puis 550°C pendant 3h).

*Préparation du catalyseur* : 1g de cendres de *Psychotria douarrei* sont prélevés. Un minimum d'HCl 12N est ajouté aux cendres (environ 20 mL) ; la totalité du solide passe en solution et prend rapidement une couleur vert pâle. Après 2h à 60°C, le mélange est évaporé à 80°C, filtré et conduit à 1g d'une poudre fine de couleur jaune pâle, couleur du dichlorure de nickel déshydraté.

*Résultats en ICP-MS (Tableau XIV)* :

Tableau XIV

| | Mg | Al | Ca | Fe | Cu | Zn | Cd | Pb | Mn | Ni |
|---|---|---|---|---|---|---|---|---|---|---|
| Cendres | 87020 | 880 | 105945 | 260 | 4740 | 7040 | 20 | 300 | 260 | 185600 |
| Catalyseur brut | 78240 | 1620 | 93719 | 1760 | 4560 | 5760 | 14 | 360 | 1160 | 270320 |

Le catalyseur brut (exemple 5.2) a été l'objet de développements en synthèse organique.

Il est très performant :

- la réaction de substitution électrophile aromatique test entre le toluène et le chlorure de benzyle (Cf mode opératoire décrit avec la montmorillonite K10, exemple 3.1.1) est de 80% après 1 heure de réaction à 20°C.
- la réaction de Diels-Alder entre le fumarate de diéthyle et le cyclopentadiène est très rapide : elle est terminée après 15 minutes d'agitation à 20°C ; ce résultat ouvre des perspectives en synthèse asymétrique. L'efficacité de la catalyse par le dichlorure de nickel et la facilité de la réaction permettent de réaliser des essais à basse température pour favoriser une induction asymétrique élevée avec le fumarate de dimenthyle.
- La réaction de Biginelli est également possible et comparable aux essais précédents. Elle est comparable à l'essai décrit dans la littérature avec du $NiCl_2$ pur et hydraté (Jun Lu, Yinjuan bai, Synthesis 2002, 4, 466).

Ces résultats sont originaux, car à l'exception de la réaction de Biginelli, $NiCl_2$ est rarement utilisé en catalyse acide de Lewis.

Un avantage de la méthode est que le traitement de la plante permet de générer différents sels de nickel à partir d'un précurseur unique : *P. douarrei.* L'intérêt est de disposer de systèmes catalytiques de solubilité différente et d'applications variées.

Les essais réussis sont les suivants :

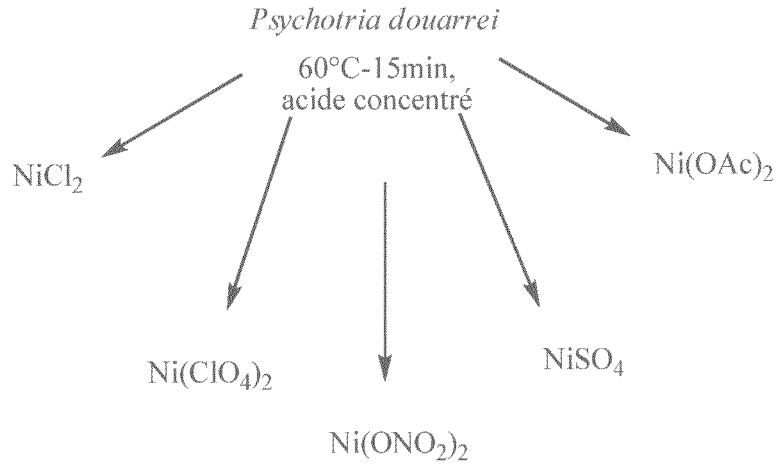


**Exemple 6: Préparation du Dichlorobis(triphenylphosphine)nickel(II), un catalyseur de couplage arynique:**

La composition de l'exemple 5.1 ($NiCl_2$, $6H_2O$) est reprise par 50 mL d'éthanol sec et chauffée à 80°C.

La triphénylphosphine (11g) est mise en solution dans 100 mL d'isopropanol sec sous atmosphère d'azote. Le mélange est agité à reflux jusqu'à dissolution totale de la triphénylphosphine. Il est ensuite additionné à la solution de dichlorure de nickel ($NiCl_2$) chaude préparée ci-dessus. La solution est agitée à reflux pendant 30 minutes puis ramenée à température ambiante.

Le mélange est filtré puis le solide résiduel est lavé par de l'éthanol froid (40mL), puis de l'éther (20mL). Le solide, dichlorobis(triphenylphosphine)nickel(II), est séché sous courant d'azote.

**Exemple 7 : Réduction du 1-phényl 2-nitroprène en 1-phényl 2-aminopropane**

Ce procédé illustre une application du procédé dans la double réduction d'une double liaison C=C et du groupe nitro.

2,5g de 1-phényl 2-nitropropène sont placés dans 25 mL d'éthanol puis ajoutés à une solution de nickel éthanolique (2g $NiCl_2$ (exemple 5.2) dans 50 ml EtOH).

1,5 mL d'acide chlorhydrique sont ajoutés lentement, puis 10,5 gramme d'aluminium sont introduits lentement. Après dissolution de l'aluminium, on ajoute alternativement 4mL d'HCl puis 0,8g d'aluminium.

Recommencer deux fois cette addition successive d'HCl et d'aluminium.

La consommation de l'aluminium est lente et nécessite 5 à 6 heures de réaction. Le milieu est ensuite neutralisé avec précaution à l'aide d'une solution aqueuse de soude. La réaction est fortement exothermique.

Au bout de 30 minutes, la phase organique devient orange, ce qui traduit la formation de l'amine attendue. Après décantation et concentration, le sirop brut obtenu est repris à l'acétone.

L'addition d'acide sulfurique fait précipiter le sulfate d'ammonium dérivé du 1-phényl 2-aminopropane, qui est isolé par filtration. Le rendement global en 1-phényl 2-aminopropane est de 65%.

**Exemple 8 : Préparation d'une composition contenant un catalyseur métallique dont le métal est Cu**

**8.1 : catalyseur issu d'*Ipomea alpina***

Le catalyseur est préparé de la même manière que pour le Zn ou le Ni, à partir d'*Ipomea alpina* (HCl 12N).

**8.2 : Catalyseur issu de *Bacopa monnieri***

<u>*Cultures et accumulation*</u> de Cu(II) ($CuSO_4$) selon S. Sinha and P. Chadra, Water, Air and Soil Pollution 51 :271-276, 1990.

<u>*Calcination*</u> : 4 plants ayant accumulé du sulfate de cuivre pendant 8 jours sont lavés abondamment (dépôt de calcaire important), séchés avec du papier filtre puis mis à l'étuve 2h à 65°. La calcination est ensuite réalisée selon le programme standard (300°C pendant 2h, puis 550°C pendant 3h).

<u>*Préparation du catalyseur*</u> : 140 mg de cendres sont prélevés. Un minimum d'HCl 1N est ajouté aux cendres (environ 2 mL) ; après une effervescence de courte durée, la quasi-totalité du solide passe en solution ; la solution s'éclaircit rapidement et devient gris-jaune, ce qui laisse supposer la formation de chlorure cuivrique. La solution est

même jaune-vert après 2h d'agitation. Après une filtration rapide, le mélange est évaporé à 80°C et conduit à 475 mg d'une poudre fine de couleur rouille (Tableau XVI) :

Tableau XVI

| Run | **Mg** | **Al** | **Ca** | **Fe** | **Cu** | **Zn** | **Cd** | **Pb** |
|---|---|---|---|---|---|---|---|---|
| | ppm | ppm | ppm | Fe | ppm | ppm | ppm | ppm |
| | | | | | | | | |
| Cat. Bacopa | 8114 | 5496 | 125880 | 5676 | 30060 | 2328 | 412 | 1578 |
| | | | | | | | | |
| | | | | | | | | |

8.3 : Hydrolyse catalysée des thiophosphates

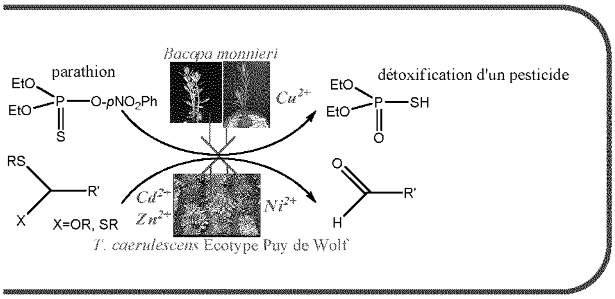


2 mL d'une solution 1 : 1 eau/éthanol à pH = 8,0 sont introduits dans un ballon de 5 mL.
140 mg de catalyseur (exemple 9.2) sont ajoutés à la solution présente.
Le mélange est agité à 40°C.
5,5 μL de parathion (conservé à 5°C) sont ajoutés au moyen d'une micro-seringue de GC à travers un septum. L'agitation est maintenue 30h à 40°C.
Le matériel souillé par le parathion (micro-seringue) est lavé avec de la soude 3 M, afin d'éliminer le parathion.
La décomposition du parathion est suivie en RMN $^{31}P$ : elle va plus vite et plus loin que sans Bacopa [$(EtO)_2P(O)O^-$ : + 20 % en 30 heures dont 12% de phosphate de diéthyle].
La réaction peut également être effectuée par un catalyseur brut issu de Thlaspi caerulescens (Ecotype Puy de Wolf) obtenu comme dans l'exemple 1.1 mais avec un rendement inférieur.

**Exemple 9 : Révélation des oximes**

Exemple 9.1

Une solution de $CuCl_2$ (exemple 9.1) à 0,5% dans l'eau est préparée et vaporisée sur un oxime préalablement déposé sur une plaque de chromatographie couche mince recouverte de silice.
Une tache vert-brun apparaît facilement. Elle est caractéristique du complexe oxime-$Cu^{2+}$.

Exemple 9.2

Une solution de $CuCl_2$ (exemple 8.2) à 0,5% dans l'eau est préparée et 2 mL de la solution obtenue sont placés dans un tube à essai (solution gris vert pâle). Quelques mg de benzaldéhyde-oxime *(E)* sont ajoutés à la solution. Après quelques secondes d'agitation, un complexe est vert foncé apparaît nettement caractéristique du complexe oxime-$Cu^{2+}$.

**Exemple 10 : Réaction de substitution électrophile aromatique par un catalyseur métallique isolé de plantes accumulant des métaux tels que Zn, Cu ou Ni.**

Le catalyseur obtenu à l'exemple 1 ($ZnCl_2$), exemple 5 ($NiCl_2$) ou exemple 8 ($CuCl_2$) est déshydraté par chauffage à 110°C, puis imprégné de montmorillonite (2g de montmorillonite pour 1,46 g de $ZnCl_2$ par exemple). Le mélange est à 110°C pendant 1 heure.

Le complexe catalytique $ZnCl_2$-montmorillonite est ajouté au mélange toluène (20 mL) et chlorure de benzyle (1,27g).

Après 1h d'agitation, le mélange est filtré et le filtrat lavé à l'hexane. Les produits de substitution électrophile isomères, les 4- et 2-méthyldiphénylméthane sont obtenus quantitativement.

**Exemple 11 : exemple comparatif d'une réaction d'halogénation d'un alcool secondaire effectuée avec une composition, contenant un catalyseur, obtenue sans filtration (étape d.)**

30,03g de feuilles déshydratées et réduites en poudre de *Thlaspi caerulescens* issues du sol de la mine des Avinières sont dosées par la méthode au zincon. Le taux de zinc présent dans la matière sèche obtenu est de 420 mg ou 2 mmoles. La matière sèche est ensuite placée dans 20 mL d'acide chlorhydrique 1N.

La solution est agitée 1 heure, puis soniquée pendant 2 heures. 1 à 2 mL d'HCl 12N sont ajoutés pour permettre une agitation satisfaisante du milieu. 2 mmoles de 4-méthyl pentan-2-ol sont additionnées directement, sans filtration, sur le mélange réactionnel précédent à 25°C. Une solution vert foncé et très hétérogène est agitée 5 heures à 40°C, un prélèvement du milieu réactionnel est placé dans quelques mL d'éther de pétrole et analysé par GC MS. Seules des traces de dérivé chloré sont observées.

## Revendications

**1.** Procédé de mise en oeuvre de réactions de synthèse organique comprenant :

a) la préparation d'une composition contenant un catalyseur métallique par

- calcination à une température comprise entre 200 et 800 °C d'une plante hyperaccumulatrice ou d'une partie de plante hyperaccumulatrice contenant au moins un métal sous forme M(II) choisi parmi le zinc (Zn) à une concentration comprise entre 2700 mg/kg et 43700 mg/kg de poids sec de plante ou de partie de plante, le nickel (Ni) à une concentration comprise entre 1000 mg/kg et 36000 mg/kg de poids sec de plante ou de partie de plante ou le cuivre (Cu) à une concentration comprise entre 1000 mg/kg et 13700 mg/kg de poids sec de plante ou de partie de plante,
- traitement à l'acide chlorhydrique aqueux des cendres ainsi obtenues,
- filtration du mélange ainsi obtenu pour obtenir un précipité et un filtrat dépourvu de chlorophylle,
- évaporation totale de l'eau du filtrat ainsi obtenu pour obtenir une composition déshydratée contenant le catalyseur métallique,

b) l'utilisation, sans rajout de métal provenant d'une autre origine que celle de ladite plante après calcination, traitement acide, filtration et sans purification ultérieure de la composition ainsi obtenue, comme catalyseur dans une réaction de synthèse organique.

**2.** Procédé selon la revendication 1 dans lequel ladite plante est choisie parmi la famille des Brassicaceae, notamment les espèces du genre *Thlaspi,* en particulier *T. caerulescens, T. goesingense, T. tatrense, T. rotundifolium, T. praecox,* les espèces du genre *Arabidopsis,* en particulier *Arabidopsis hallerii,* et du genre *Alyssum,* en particulier *A. bertolonii, A. serpyllifolium,* des Fabaceae, en particulier *Anthyllis vulneraria,* des Sapotaceae, notamment les espèces *Sebertia acuminata, Planchonella oxyedra,* des Convolvulaceae, notamment les espèces *Ipomea alpina, Planchonella oxyedra* ou des Rubiaceae, notamment les espèces *Psychotria douarrei,* en particulier *P. costivenia, P. clementis, P. vanhermanii,* ou des Scrophulariaceae, notamment les espèces du genre *Bacopa,* en particulier *Bacopa monnieri,* les algues, en particulier les algues rouges, notamment les Rhodophytes, notamment *rhodophyta bostrychia,* les algues vertes ou les algues brunes.

**3.** Procédé selon l'une des revendications 1 à 2, dans lequel ladite plante appartient à la famille des Brassicacea, notamment *Thlaspi caerulescens* ou *Arabidopsis hallerii,* et le métal accumulé par ladite plante est Zn.

4. Procédé selon la revendication 1, dans lequel ladite composition comprend de plus au moins l'un des métaux suivants: Mg, Ca, Fe(III), Al(III), Cu, Cd, Pb.

5. Procédé selon l'une des revendications 1 ou 2, dans lequel ladite plante est une Sapotaceae, en particulier *Sebertia acuminata,* une Rubiaceae, en particulier *Psychotria douarrei,* ou une Brassicaceae, en particulier *Thlaspi goesingense* ou *Thlaspi caerulescens,* et le métal accumulé par ladite plante est Ni.

6. Procédé selon l'une des revendications 1 ou 2, dans lequel ladite plante est une Convolvulaceae, notamment *Ipomea alpina* ou une Brassicaceae, en particulier *Thlaspi caerulescens,* ou une Scrophulariaceae, en particulier *Bacopa monnieri* et le métal accumulé par ladite plante est Cu.

7. Procédé selon la revendication 1, dans lequel ladite plante appartient à la famille des Brassicacea, notamment *Thlaspi caerulescens* ou *Arabidopsis hallerii,* ledit acide est HCl 1N et le métal de ladite composition est le Zn et comprend éventuellement au moins un métal choisi parmi Mg, Ca, Fe(III), Al(III), Cu, Cd, Pb, Na(I), Mn, Ni.

8. Procédé selon la revendication 1, dans lequel ladite plante appartient à la famille des Brassicacea, notamment *Thlaspi caerulescens* ou *Arabidopsis hallerii,* ledit acide est HCl 12N et le métal de ladite composition est le Zn et comprend éventuellement au moins un métal choisi parmi Mg, Ca, Fe(III), Al(III), Cu, Cd, Pb.

## Patentansprüche

1. Verfahren zum Durchführen von organischen Synthesereaktionen, aufweisend:

   a) Herstellen einer Zusammensetzung, die einen Metallkatalysator aufweist, mittels

   - Kalzinierens einer hyperakkumulativen Pflanze oder eines Teils einer hyperakkumulativen Pflanze, die mindestens ein Metall in Form von M(II) aufweist, ausgewählt aus Zink (Zn) in einer Konzentration zwischen einschließlich 2700 mg/kg und 43700 mg/kg Trockengewicht der Pflanze oder des Pflanzenteils, Nickel (Ni) in einer Konzentration zwischen einschließlich 1000 mg/kg und 36000 mg/kg Trockengewicht der Pflanze oder des Pflanzenteils oder Kupfer (Cu) in einer Konzentration zwischen einschließlich 1000 mg/kg und 13700 mg/kg Trockengewicht der Pflanze oder des Pflanzenteils bei einer Temperatur zwischen einschließlich 200 und 800°C;
   - Behandelns der so erhaltenen Aschen mit wässriger Salzsäure,
   - Filtrierens der so erhaltenen Mischung zum Erhalten eines Niederschlags und eines chlorophyllfreien Filtrats,
   - vollständigen Verdampfens des Wassers aus dem so erhaltenen Filtrat zum Erhalten einer dehydrierten Zusammensetzung, die den Metallkatalysator aufweist,

   b) das Verwenden als Katalysator in einer organischen Synthesereaktion ohne Zusatz von Metall aus einem anderen Ursprung als dem der Pflanze nach dem Kalzinieren, der Säurebehandlung, der Filtration und ohne weitere Reinigung der so erhaltenen Zusammensetzung.

2. Verfahren gemäß Anspruch 1, wobei die Pflanze ausgewählt ist aus der Familie der Brassicaceae, insbesondere der Arten der Gattung *Thlaspi,* insbesondere *T. caerulescens, T. goesingense, T. tatrense, T. rotundifolium, T. praecox,* der Arten der Gattung *Arabidopsis,* insbesondere *Arabidopsis hallerii,* und der Art *Alyssum,* insbesondere A. *bertolonii, A. serpyllifolium,* der Fabaceae, insbesondere *Anthyllis vulneraria,* der Sapotaceae, insbesondere die Arten *Sebertia acuminata, Planchonella oxyedra,* der Convolvulaceae, insbesondere die Arten *Ipomea alpina, Planchonella oxyedra* oder der Rubiaceae, insbesondere die Arten *Psychotria douarrei,* insbesondere *P. costivenia, P. clementis, P. vanhermanii,* oder der Scrophulariaceae, insbesondere Arten der Gattung *Bacopa,* insbesondere *Bacopa monnieri,* Algen, insbesondere Rotalgen, insbesondere Rhodophyten, insbesondere *Rhodophyta bostrychia,* Grünalgen oder Braunalgen.

3. Verfahren gemäß einem der Ansprüche 1 bis 2, wobei die Pflanze zur Familie der Brassicacea gehört, insbesondere *Thlaspi caerulescens* oder *Arabidopsis hallerii,* und das von der Pflanze akkumulierte Metall Zn ist.

4. Verfahren gemäß Anspruch 1, wobei die Zusammensetzung ferner mindestens eines der folgenden Metalle aufweist: Mg, Ca, Fe(III), Al(III), Cu, Cd, Pb.

5. Verfahren gemäß einem der Ansprüche 1 oder 2, wobei die Pflanze eine Sapotaceae, insbesondere *Sebertia acuminata,* eine Rubiaceae, insbesondere *Psychotria douarrei,* oder eine Brassicaceae, insbesondere *Thlaspi goesingense* oder *Thlaspi caerulescens* ist, und das von der Pflanze akkumulierte Metall Ni ist.

6. Verfahren gemäß einem der Ansprüche 1 oder 2, wobei die Pflanze eine Convolvulaceae, insbesondere *Ipomea alpina* oder eine Brassicaceae, insbesondere *Thlaspi caerulescens,* oder eine Scrophulariaceae, insbesondere *Bacopa monnieri* ist und das von der Pflanze akkumulierte Metall Cu ist.

7. Verfahren gemäß Anspruch 1, wobei die Pflanze zur Familie der Brassicacea gehört, insbesondere *Thlaspi caerulescens* oder *Arabidopsis hallerii,* die Säure 1N HCl ist und das Metall der Zusammensetzung Zn ist und optional mindestens ein Metall, ausgewählt aus Mg, Ca, Fe(III), Al(III), Cu, Cd, Pb, Na(I), Mn, Ni aufweist.

8. Verfahren gemäß Anspruch 1, wobei die Pflanze zur Familie der Brassicacea gehört, insbesondere *Thlaspi caerulescens* oder *Arabidopsis hallerii,* die Säure 12N HCl ist und das Metall der Zusammensetzung Zn ist und optional mindestens ein Metall, ausgewählt aus Mg, Ca, Fe(III), Al(III), Cu, Cd, Pb aufweist.

## Claims

1. Method for performing organic synthesis reactions, comprising:

   a) preparation of a composition comprising a metal catalyst by

   - calcination at a temperature of between 200 and 800°C of a hyperaccumulating plant or of a hyperaccumulating plant part containing at least one metal in M(II) form chosen from zinc (Zn) at a concentration of between 2700 mg/kg and 43,700 mg/kg of dry weight of plant or plant part, nickel (Ni) at a concentration of between 1000 mg/kg and 36,000 mg/kg of dry weight of plant or plant part, or copper (Cu) at a concentration of between 1000 mg/kg and 13,700 mg/kg of dry weight of plant or plant part,
   - treatment with aqueous hydrochloric acid of the ashes so obtained,
   - filtration of the mixture so obtained in order to obtain a precipitate and a filtrate free of chlorophyll,
   - total evaporation of the water from the filtrate so obtained in order to obtain a dehydrated composition comprising the metal catalyst,

   b) use, without the addition of metal derived from a source other than that of said plant after calcination, acid treatment, filtration and without subsequent purification of the composition so obtained, as catalyst in an organic synthesis reaction.

2. Method according to claim 1, wherein said plant is chosen from the family of the Brassicaceae, especially the species of the genus *Thlaspi,* in particular *T. caerulescens, T. goesingense, T. tatrense, T. rotundifolium, T. praecox,* the species of the genus *Arabidopsis,* in particular *Arabidopsis hallerii,* and of the genus *Alyssum,* in particular *A. bertolonii, A. serpyllifolium,* of the Fabaceae, in particular *Anthyllis vulneraria,* of the Sapotaceae, especially the species *Sebertia acuminata, Planchonella oxyedra,* of the Convolvulaceae, especially the species *Ipomea alpina, Planchonella oxyedra* or of the Rubiaceae, especially the species *Psychotria douarrei,* in particular *P. costivenia, P. clementis, P. vanhermanii,* or of the Scrophulariaceae, especially the species of the genus *Bacopa,* in particular *Bacopa monnieri,* the algae, in particular the red algae, especially the Rhodophytes, especially *Rhodophyta bostrychia,* the green algae or the brown algae.

3. Method according to either claim 1 or claim 2, wherein said plant belongs to the family of the Brassicacea, especially *Thlaspi caerulescens* or *Arabidopsis hallerii,* and the metal accumulated by said plant is Zn.

4. Method according to claim 1, wherein said composition additionally comprises at least one of the following metals: Mg, Ca, Fe(III), Al(III), Cu, Cd, Pb.

5. Method according to either claim 1 or claim 2, wherein said plant is a Sapotaceae, in particular *Sebertia acuminata,* a Rubiaceae, in particular *Psychotria douarrei,* or a Brassicaceae, in particular *Thlaspi goesingense* or *Thlaspi caerulescens,* and the metal accumulated by said plant is Ni.

6. Method according to either claim 1 or claim 2, wherein said plant is a Convolvulaceae, especially *Ipomea alpina,* or

a Brassicaceae, in particular *Thlaspi caerulescens,* or a Scrophulariaceae, in particular *Bacopa monnieri,* and the metal accumulated by said plant is Cu.

**7.** Method according to claim 1, wherein said plant belongs to the family of the Brassicacea, especially *Thlaspi caerulescens* or *Arabidopsis hallerii,* said acid is 1N HCl and the metal of said composition is Zn and optionally comprises at least one metal chosen from Mg, Ca, Fe(III), Al(III), Cu, Cd, Pb, Na(I), Mn, Ni.

**8.** Method according to claim 1, wherein said plant belongs to the family of the Brassicacea, especially *Thlaspi caerulescens* or *Arabidopsis hallerii,* said acid is 2N HCl and the metal of said composition is Zn and optionally comprises at least one metal chosen from Mg, Ca, Fe(III), Al(III), Cu, Cd, Pb.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Phytoremediation of contaminated soil in water. Lewis Publishers, 2000
- **FRÉROT et al.** Specific interactions between local metallicolous plants improve the phytostabilazation of mine soils. *Plant and Soil,* 2006, vol. 282, 53-65
- **FRÉROT et al.** Specific interactions between local metallicolous plants improve the phytostabilisation of mine soils. *Plant and Soil,* 2006, vol. 282, 53-65
- **VIDAL et al.** Mesorhizobium metallidurans sp. nov., a novel metal-resistant symbiont of Anthyllis vulneraria, growing on metallicolous soil in Languedoc. *International Journal of Systematic and Evolutionary Microbiology,* 2009
- **DARCY M.** *Métallurgie du zinc,* 1988
- **PHILIBERT J. et al.** Métallurgie du minerai au matériau. 2002
- *Biorecovery,* vol. 1, 81-126
- **REEVES, R. D. ; BROOKS, R. R.** European species of Thlaspi L. (Cruciferae) as indicators of nickel and zinc. *J. Geochem. Explor.,* 1983, vol. 18, 275-283
- **REEVES, R. D. ; BROOKS, R. R.** Hyperaccumulation of lead and zinc by two metallophytes from a mining area in Central Europe. *Environ. Pollut.,* 1983, vol. 31, 277-287
- **BÄUML, E. ; TSCHESCHLOK, K. ; POCK, R. ; MAYR, H.** Synthesis of γ-lactones from alkenes employing p-methoxybenzyl chloride as CH2---CO-2 equivalent. *Tetrahedron Lett.,* 1978, vol. 29, 6925-6926
- **C. GRISON ; J. ESCARRE ; M.L. BERTHOMME ; J. COUHET-GUICHOT ; C. GRISON ; F. HOSY.** *Actualité Chimique,* 2010, vol. 340, 27-32
- **MACNAIR ; SMIRNOFF.** *Commun. Soil Sci. Plant Anal.,* 1999, vol. 30, 1127-1136
- **JUN LU ; YINJUAN BAI.** *Synthesis,* 2002, vol. 4, 466
- **S. SINHA ; P. CHADRA.** *Water, Air and Soil Pollution,* 1990, vol. 51, 271-276